# EUROPEAN PATENT SPECIFICATION

(11) **EP 0 498 665 B1**
(45) Date of publication and mention of the grant of the patent: **24.04.1996**
(21) Application number: 92301050.8
(22) Date of filing: 07.02.1992
(51) Int. Cl.: C07C 259/06, C07C 317/44, C07C 323/52, C07D 333/34, A61K 31/165, C07C 237/22

(54) **Hydroxamic acids derivatives, process for their preparation and use thereof**
Hydraxamsäurederivate, Verfahren zu ihrer Herstellung und ihre Verwendung
Dérivés de l'acide hydroxamique, procédé pour leur préparation et leur utilisation

(30) Priority: 07.02.1991 GB 9102635
(43) Date of publication of application: 12.08.1992
(73) Proprietor: BRITISH BIOTECH PHARMACEUTICALS LIMITED, Cowley Oxford, OX4 5LY (GB)
(72) Inventor: Beckett, Raymond Paul, Aston, Oxon, OX18 2EB (GB); Crimmin, Michael John, Marlow Bottom, Bucks, HP18 9JA (GB); Davidson, Alan Horsby, Witney, Oxon, OX8 6RH (GB)
(74) Representative: Walls, Alan James

(56) References cited:
- EP-A- 0 214 639
- EP-A- 0 236 872
- EP-A- 0 274 453
- WO-A-90/05716
- WO-A-90/05719

## Description

A number of small peptide like compounds which inhibit metalloproteinase have been described. Perhaps the most notable of these are those relating to the angiotensin converting enzyme (ACE) where such agents act to blockade the conversion of the decapeptide angiotensin I to angiotensin II, a potent pressor substance. Compounds of this type are described in EP-A-0012401.

Certain hydroxamic acids have been suggested as collagenase inhibitor as in US-A-4 599 361, WO-A-9005716 and WO-A-9005719. Other hydroxamic acids have been prepared as ACE inhibitors, for example, in US-A-4,105,789, while still others have been described as enkephalinase inhibitors as in US-A-4,495,540.

EP-A-0214639 relates to hydroxamic acid based collagenase inhibitors of the formula: 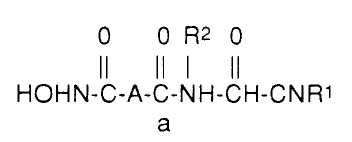
wherein R¹ is C₁-C₆ alkyl; R² is C₁-C₆ alkyl, benzyl, hydroxybenzyl, benzyloxybenzyl, (C₁-C₆ alkoxy)benzyl, or benzyloxy(C₁-C₆ alkyl); a is a chiral center with optional R or S stereochemistry; A is a 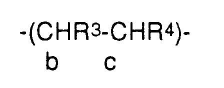
group or a -(CR³=CR⁴)- group wherein b and c are chiral centers with optional R or S stereochemistry; R³ is hydrogen, C₁-C₆ alkyl, phenyl, or phenyl (C₁-C₆ alkyl); and R⁴ is hydrogen or C₁-C₆ alkyl, phenyl (C₁-C₆ alkyl), cycloalkyl, or cycloalkyl (C₁-C₆ alkyl).

WO-A-9005719 relates to hydroxamic acid based collagenase inhibitors, which also have stromelysin inhibitors activity, and which differ in structure from those of EP-A-0214639 principally in the identity of the substituent corresponding to R³ of EP-A-0214639. In WO-A-9005719 that substituent is a substituted mercaptoalkyl group.

It would be desirable if alternative collagenase inhibitors were made available, having different or improved properties.

The hydroxamic acids of the current invention act as inhibitors of mammalian collagenase which initiates collagen breakdown. There is evidence implicating collagenase as one of the key enzymes in the breakdown of articular cartilage and bone in rheumatoid arthritis (Arthritis and Rheumatism, **20**, 1231-1239, (1977). Potent inhibitors of collagenase are useful in the treatment of rheumatoid arthritis and related diseases in which collagenolytic activity is important. These diseases include corneal ulceration, osteoporosis, periodontitis, gingivitis and tumour invasion.

The current invention relates to a series of hydroxamic acids, which act as inhibitors of metalloproteinase, their preparation and pharmaceutical compositions containing them.

In a first aspect of the invention there is provided a compound of general formula (I): 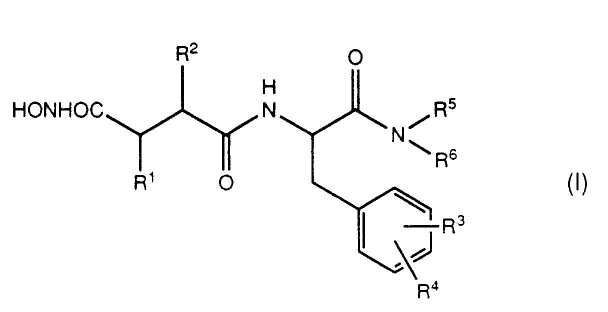
wherein
R¹ is hydrogen; C₁-C₆ alkyl; phenyl; phenyl substituted by up to four substituents each of which may independently be C₁-C₆ alkyl, C₁-C₆ alkoxy, hydroxy, thiol, C₁-C₆ alkylthiol, amino, halo, trifluoromethyl, nitro, -COOH, -COONH₂, or -CONHR^{A} wherein R^{A} represents a C₁-C₆ alkyl group or the residue of an amino acid selected from alanine, valine, leucine, isoleucine, phenylalanine, tryptophan, methionine, glycine, serine, threonine, cysteine, tyrosine, asparagine, glutamine, aspartic acid, glutaminic acid and histidine; phenyl (C₁-C₆ alkyl) or heterocyclyl;
or R¹ is -ASOₙR⁷, wherein A represents a C₁-C₆ hydrocarbon chain optionally substituted with one or more C₁-C₆ alkyl groups, phenyl groups, or phenyl groups substituted by up to four substituents as specified above; n = 0, 1 or 2; and R⁷ is C₁-C₆ alkyl; phenyl; phenyl substituted by up to four substituents as specified above; phenyl (C₁-C₆ alkyl, heterocyclyl, (C₁-C₆ alkyl)acyl, thienyl or phenacyl:
R² is hydrogen, C₁-C₆ alkyl, C₂-C₆ alkenyl phenyl(C₁-C₆ alkyl), or C₃-C₈ cycloalkyl(C₁-C₆ alkyl);
R⁵ is hydrogen or C₁-C₆ alkyl, or (C₁-C₆ alkyl)phenyl;
R⁶ is hydrogen or methyl;
characterised in that
R⁴ is hydrogen and
R³ is -O-CH₂-CO-R⁸ where R⁸ is hydroxyl; (C₁-C₆)alkoxy; phenyl (C₁-C₆)alkoxy; amino; (C₁-C₆)alkylamino; di((C₁-C₆)alkyl)amino; phenyl (C₁-C₆)alkylamino; the residue of an amino acid or acid halide, ester or amide derivative thereof, said residue being linked via an amide bond, said amino acid being selected from glycine, α or β alanine, valine, leucine, isoleucine, phenylalanine, tyrosine, tryptophan serine, threonine, cysteine, methionine, asparagine, glutamine, lysine, histidine, arginine, glutamic acid, and aspartic acid;
or a salt thereof.

Hereafter in this specification the term "compound" includes salt unless the context requires otherwise.

As used herein the term "C₁-C₆ alkyl" refers to a straight or branched chain alkyl moiety having from one to six carbon atoms, including for example, methyl, ethyl, propyl, isopropyl, butyl, t-butyl, pentyl, hexyl and the like.

The term "C₂-C₆ alkenyl" refers to a straight or branched chain alkyl moiety having two to six carbons and having in addition one double bond, of either E or Z stereochemistry where applicable. This term would include, for example, vinyl, 1-propenyl, 1- and 2-butenyl, 2-methyl-2-propenyl etc.

The term "cycloalkyl" refers to a saturated alicyclic moiety having from 3 to 8 carbon atoms and includes for example, cyclopropyl, cyclobutyl, cyclopentyl, cyclohexyl and the like.

The term 'heterocyclyl' refers to a saturated or unsaturated ring containing at least one hetero atom such as nitrogen, oxygen or sulphur and includes for example, furan, pyrrole, thiophene, morpholine, pyridine, dioxane, imidazoline, pyrimidine and pyridazine.

The term "substituted", as applied to a phenyl or other aromatic ring, means substituted with up to four substituents each of which independently may be C₁-C₆ alkyl, C₁-C₆ alkoxy, hydroxy, thiol, C₁-C₆ alkylthiol, amino, halo (including fluoro, chloro, bromo and iodo), trifluoromethyl, nitro, -COOH, -COONH₂ or -CONHR^{A}, wherein R^{A} represents a C₁-C₆ alkyl group or the characteristic side chain of an amino acid such as alanine, valine, leucine, isoleucine, phenylalanine, tryptophan, methionine, glycine, serine, threonine, cysteine, tyrosine, asparagine, glutamine, aspartic acid, glutamic acid, lysine, arginine or histidine.

The term "amino acid" means one of the following R or S amino acids: glycine, alanine, valine, leucine, isoleucine, phenylalanine, tyrosine, tryptophan, serine, threonine, cysteine, methionine, asparagine, glutamine, lysine, histidine, arginine, glutamic acid and aspartic acid.

Derivatives of amino acids include halides, esters and substituted or unsubstituted amides, for example N methyl amide.

There are several chiral centres in the compounds according to the invention because of the presence of asymmetric carbon atoms.

The presence of several asymmetric carbon atoms gives rise to a number of diastereomers with the appropriate R or S stereochemistry at each chiral centre. The invention is understood to include all such diastereomers and mixtures thereof.

Preferred compounds include those in which, independently or in combination :
R¹ represents a hydrogen atom or a C₁-C₄ alkyl, or phenyl group; or
R¹ represents ASOₙR⁷ in which A is C₁-C₄ hydrocarbon chain alkyl (for example methylene), n = 0, and R⁷ is a phenyl, substituted phenyl or thienyl group;
R² represents a C₁-C₅ alkyl (for example isobutyl) group;
R⁵ represents a C₁-C₄ alkyl (for example methyl) group;
R⁶ represents a hydrogen atom.

Particularly preferred compounds include :
[4-(N-Hydroxamino)-2R-isobutylsuccinyl]-L-(4-oxymethylcarboxylic acid)phenylalanine-N-methylamide;
[4-(N-Hydroxamino)-2R-isobutylsuccinyl]-L-(4-oxymethylcarboxy-N-methylamide)-phenylalanine-N-methylamide;
[4-(N-Hydroxamino)-2R-isobutylsuccinyl]-L-(4-oxymethylcarboxy-beta-alanine)-phenylalanine-N-methylamide;
[4-(N-Hydroxamino)-2R-isobutylsuccinyl]-L-(4-oxymethylcarboxyglycine)-phenylalanine-N-methylamide;
[4-(N-Hydroxamino)-2R-isobutylsuccinyl]-L-(4-oxymethylcarboxy-N-benzylamide)-phenylalanine-N-methylamide;
[4-(N-Hydroxamino)-2R-isobutyl-3S-(2-thienylthiomethyl)succinyl]-L-(4-oxymethylcarboxymethyl)-phenylalanine-N-methylamide;
[4-(N-Hydroxamino)-2R-isobutyl-3S-(2-thienylthiomethyl)succinyl]-L-(4-oxymethylcarboxylic acid)-phenylalanine-N-methylamide;
[4-(N-Hydroxamino)-2R-isobutyl-3S-(2-thienylthiomethyl)succinyl]-L-(4-oxymethylcarboxyglycyl methyl ester)-phenylalanine-N-methylamide;
[4-(N-Hydroxamino)-2R-isobutyl-3S-(2-thienylthiomethyl)succinyl]-L-(4-oxymethylcarboxyglycine)-phenylalanine-N-methylamide;
[4-(N-Hydroxamino)-2R-isobutyl-3S-methylsuccinyl]-L-(4-oxymethylcarboxyglycyl methyl ester)-phenylalanine-N-methylamide; and
[4-(N-Hydroxamino)-2R-isobutyl-3S-methylsuccinyl]-L-(4-oxymethylcarboxyglycine)-phenylalanine-N-methylamide;
or a salt of one of them.

Compounds of the general formula (I) may be prepared by any suitable method known in the art and/or by the following process, which itself forms part of the invention.

According to a second aspect of the invention, there is provided a process for preparing a compound of general formula (I) as defined above, the process comprising:
(a) deprotecting (for example by hydrogenation) a compound of general formula II 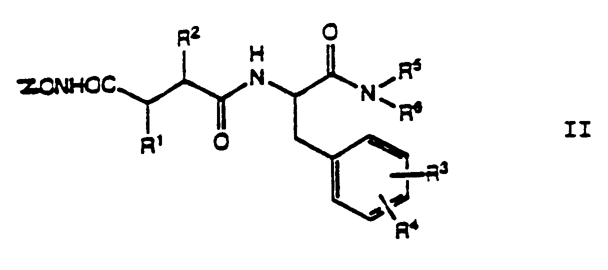 Wherein:
   R¹, R², R³, R⁴, R⁵ and R⁶ are as defined in the general formula I and Z represents a suitable protective group (e.g. tert-butyl, tertbutylsilyl, benzyl or substituted benzyl);
(b) reacting a compound of general formula III 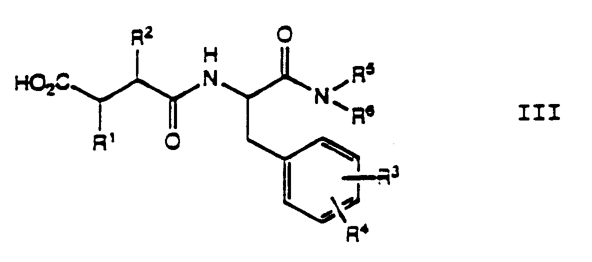 Wherein:
   R¹, R², R³, R⁴, R⁵, and R⁶ are as defined in the general formula I,
   with hydroxylamine or a salt thereof; and
(c) optionally after step (a) or step (b) converting a compound of general formula I into another compound of general formula I.

Compounds of general formula I which are sulphoxide or sulphones can be derived from thio compounds of general formula I by oxidation. Alternatively, compounds of general formula II, or III which contain sulphur can be oxidised.

A compound of general formula II can be obtained by coupling, for example by conventional coupling techniques, a compound of general formula III with an O-protected hydroxylamine of formula NH₂OZ; wherein Z is as defined in general formula II.

A compound of general formula III can be prepared by
(a) de-esterifying (for example under acid or base catalysis) a compound of general formula IV 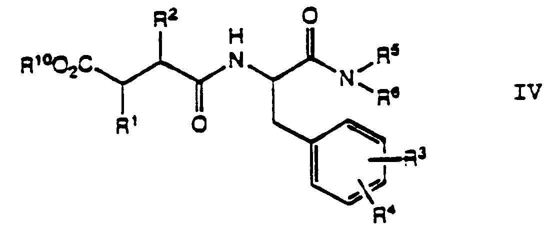 Wherein:
   R¹, R², R³, R⁴, R⁵, and R⁶ are as defined in the general formula I, and R¹⁰ represents a C₁ -C₆ alkyl or benzyl group; or
(b) by reacting a compound of general formula V 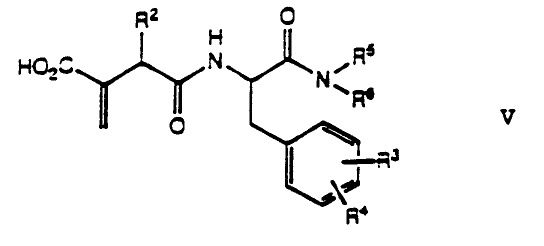 Wherein:
   R², R³, R⁴, R⁵, and R⁶ are as defined in the general formula I,
   either with a thiol of general formula R⁷SH, wherein R⁷ is as defined in general formula I, to give a compound of general formula III in which R¹ is ASOₙR⁷, A represents a methylene group and n is 0.
   or with compound R¹X where R¹ is benzyl or substituted benzyl and X is F, Cl, Br of I in the presence of a palladium catalyst to provide a compound of general formula VI 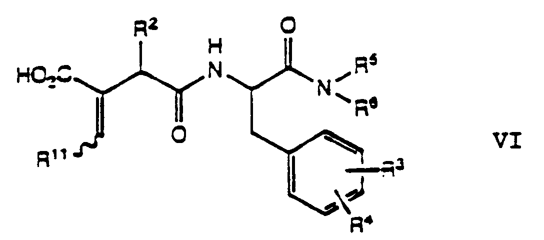 Wherein:
   R², R³, R⁴, R⁵, and R⁶ are as defined in the general formula I, and R¹ is benzyl or substituted benzyl, which may be converted to a compound of general formula III wherein R² is benzyl or substituted benzyl, by hydrogenation; or
(c) by converting a compound of general formula III into another compound of general formula III.

A compound of general formula V can be prepared from a compound of general formula IV 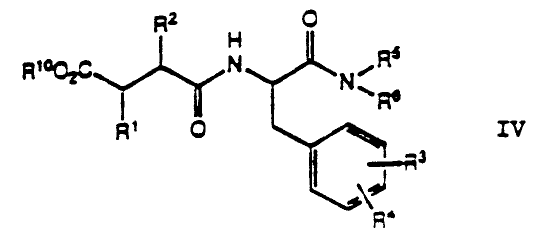
Wherein:
R², R³, R⁴, R⁵, and R⁶ are as defined in the general formula I, R¹ is carboxybenzyl or carboxy (C₁ - C₆) alkyl and R10 is benzyl or (C₁ -C₆) alkyl, and R¹⁰ is benzyl or (C₁ - C₆) alkyl,
by de-esterification (for example by hydrogenation) followed by reaction with formaldehyde in the presence of morpholine.

A compound of general formula IV can be prepared
(a) By reacting, for example by conventional coupling techniques, an acid of formula VII, or an activated ester derivative thereof, 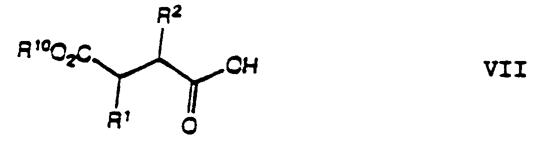 Wherein:
   R¹ and R² are as defined in the general formula I, and R¹⁰ is as defined above.
   with an amine of general formula VIII 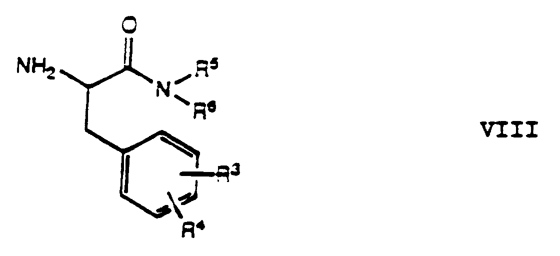 Wherein:
   R³, R⁴, R⁵, and R⁶ are as defined in the general formula I;
(b) by converting a compound of general formula IV into another compound of general formula IV.

An amine of general formula VIII can be prepared by deprotection (for example with trifluoroacetic acid) of a compound of general formula IX 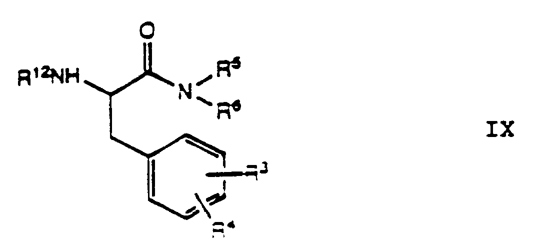
Wherein:
R¹² is a conventional amine protecting group and R³, R⁴, R⁵ and R⁶ are as defined in general formula I.

A compound of general formula IX may be prepared by coupling an acid of general formula X 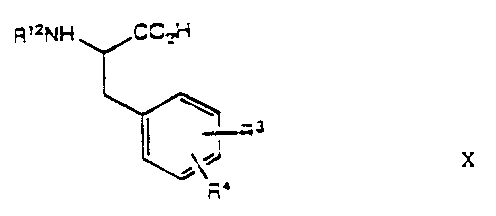
Wherein:
R³ and R⁴ , are defined as in general formula I, with an amine of general formula XI 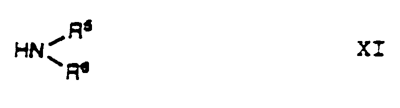
Wherein:
R⁵ and R⁶ are as defined in general formula I
A compound of general formula X may be prepared
(a) by reaction of an aryl halide of general formula XII 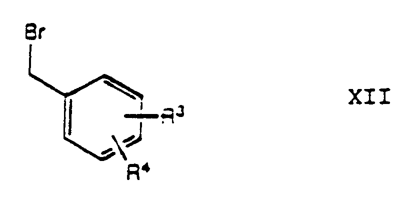 Wherein R3 and R4 are as defined in general formula I with a glycinate anion equivalent of formula XIII 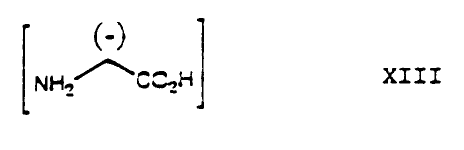 followed by acid hydrolysis, protection of the amino function and base catalysed release of the carboxylic acid; or
(b) by converting a compound of general formula X to another compound of general formula X.

A compound of general formula VII may be prepared by reaction of a compound of general formula XIV 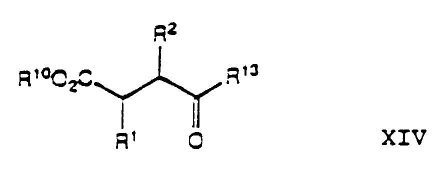
Wherein:
R² is as defined in the general formula I, R¹ is hydrogen, R¹⁰ is as described above and R¹³ is a chiral auxiliary for example as described by Evans (J. Amer. Chem. Soc., **104**, 1737, (1982)).
with lithium hydroxide/hydrogen peroxide.

A compound of general formula XIV may be produced by alkylation of the anion of a compound of general formula XV 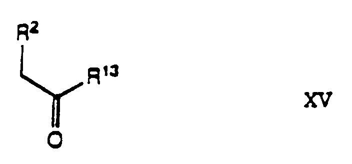
Wherein:
R² is as defined in the general formula I and R¹³ is a chiral auxiliary,
with an alkylating agent of general formula XVI. 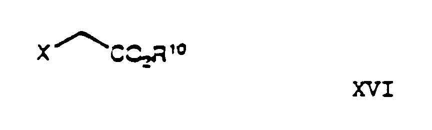
Wherein:
R¹⁰ is as described above and X is a leaving group, for example bromide, iodide or triflate.

Compounds of general formulae XI, XII, XIII, XV and XVI and other reagents are either available commercially or can be synthesised by simple chemical procedures.

The potency of compounds of the present invention to act as inhibitors of collagenase was determined by the procedure of Cawston and Barrett, (Anal. Biochem., 99, 340 -345, 1979) whereby a 1mM solution of the inhibitor being tested or dilutions thereof is incubated at 37°C for 16 hours with collagen and collagenase (buffered with Tris HCl - CaCl₂; pH 7.6). The collagen is acetylated ¹⁴C collagen prepared by the method of Cawston and Murphy (Methods in Enzymology, **80**, 711, (1981)). The samples are centrifuged to sediment undigested collagen and an aliquot of the radioactive supernatant removed for assay on scintillation counter as a measure of hydrolysis. The collagenase activity in the presence of 1 mM inhibitor, or a dilution thereof, is compared to activity in a control devoid of inhibitor and the results reported as that inhibitor concentration effecting 50% inhibition of the collagenase.

In a further aspect of the invention there is provided the use of a compound of general formula I in medicine, particularly in a method of treatment of diseases in which collagenolytic activity is important.

In another aspect of the invention there is provided the use of a compound of general formula I in the preparation of an agent for the treatment of diseases in which collagenolytic activity is important.

The invention also provides a pharmaceutical composition comprising one or more compounds of general formula I in association with one or more non-toxic pharmaceutically acceptable carriers and/or diluents and/or adjuvants. Other active ingredients may also be included in the compositions of the invention.

The compositions of the present invention may be formulated for administration by any route depending on the disease being treated. The compositions may be in the form of tablets, capsules, powders, granules, lozenges, liquid or gel preparations, such as oral, topical, or sterile parental solutions or suspensions.

Tablets and capsules for oral administration may be in unit dose presentation form, and may contain conventional excipients. Examples of these are banding agents such as syrup, acacia, gelatin, sorbitol, tragacanth, and polyvinylpyrollidone; fillers for example lactose, sugar, maize-starch, calcium phosphate, sorbitol or glycine; tabletting lubricants, for example magnesium sterate, talc, polyethylene glycol or silica; disintegrants, for example potato starch, or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatin, hydrogenated edible fats; emulsifiying agents, for example lecithin, sorbitan monooleate, or acacia; non-aqueous vehicles (which may include edible oils), for example almond oil, fractionated coconut oil, oily esters such as glycerine, propylene glycol, or ethyl alcohol; preservatives, for example methyl or propyl p-hydroxybenzoate or sorbic acid, and if desired conventional flavouring or colouring agents.

The dosage unit involved in oral administration may contain from about 1 to 250 mg, preferably from about 25 to 250 mg. A suitable daily dose for a mammal amy vary widely depending on the condition of the patient. However, a dose of about 0.1 to 300mg/kg body weight, particularly from about 1 to 100 mg/kg body weight may be appropriate.

For topical application to the skin the drug may be made up into a cream, lotion or ointment. Cream or ointment formulations that may be used for the drug are conventional formulations well known in the art, for example, as described in standard text books of pharmaceutics such as the British Pharmacopoeia.

For topical applications to the eye, the drug may be made up into a solution or suspension in a suitable sterile aqueous or non-aqueous vehicle. Additives, for instance buffers such as sodium metabisulphite or disodium edeate; preservatives including bactericidal and fungicidal agents, such as phenyl mercuric acetate or nitrate, benzalkonium chloride or chlorohexidine, and thickening agents such as hypromellose may also be included.

The dosage employed for the topical administration will, of course, depend on the size of the area being treated. For the eyes each dose will be typically in the range from 10 to 100 mg of the drug.

The active ingredient may also be administered parenterally in a sterile medium. The drug depending on the vehicle and concentration used, can either be suspended or dissolved in the vehicle. Advantageously, adjuvants such as local anaesthetic, preservatives and buffering agents can be dissolved in the vehicle.

For use in the treatment of rheumatoid arthritis the compounds of this invention can be administered by the oral route or by injection intra-articularly into the affected joint. The daily dosage for a 70 kg mammal will be in the range of 10 mgs to 1 gram.

The following examples illustrate the invention, but are not intended to limit the scope in any way.

The following abbreviations have been used in the Examples:
- DCM -: Dichloromethane
- DMF -: N,N-Dimethylformamide
- HOBT -: Hydroxybenztriazole
- NMM -: N-Methylmorpholine
- TFA -: Trifluoroacetic acid
- THF -: Tetrahydrofuran
- WSCDI -: N-(Dimethylaminoethyl)-N'-ethylcarbodiimide.

### EXAMPLES

### Example 1

### [4-(N-Hydroxyamino)-2R-isobutylsuccinyl]-L-(4-oxymethylcarboxylic acid)-phenylalanine-N-methylamide

### Example 1a

### N-(4-Methylpentanoyl)-4S-phenylmethyl-2-oxazolidinone

A dry 500 ml flask equipped with a magnetic stirrer was charged with (S)-4-Phenylmethyl-2-oxazolidinone (17.72g, 0.1 mol), this was capped with a rubber septum and flushed with N₂. Anhydrous THF (300 ml) was added via cannula and the resulting solution was cooled to -78°C in an acetone/dry ice bath. A solution of 1.47M n-butyllithium in hexane (68.4 ml, 0.101 mol) was transferred via cannula to a dry, septum-stoppered 100 ml dropping funnel. This was added dropwise to the THF solution over 10 minutes.

4-methyl valeric acid chloride (14.80g 0.11 mol) was added in one portion by syringe after completion of the addition of n-butyllithium. The resulting solution was stirred at -78°C for 30 minutes and then allowed to warm to ambient temperature over 30 minutes. Excess of the acid chloride was quenched by the addition of aq. NH₄Cl (60 ml) and the bulk of the solvent was removed. The resulting slurry was extracted with dichloromethane (2 x 80 ml). The combined organic extracts were washed with 1M NaOH (75 ml), brine (75 ml), dried (Na₂SO₄ anhyd.) and filtered. The solvent was removed to yield a yellow oil (29.20g, 106%).

Analysis calculated for C₁₆H₂₁NO₃ MWt = 275.34
delta_{H} (250 MHz, CDCl₃), 0.97 (6H, d, C(CH₃)2, J=6.2 Hz), 1.53-1.76 (3H, m, CH₂CHMe₂), 2.78 (1H, dd, CH₂Ph, J=9.5 Hz), 2.85-3.05 (2H, m, COCH₂) 3.30 (1H, dd, CH₂Ph, J=3.3 Hz) 4.16-4.25 (2H, m, CH₂OCO) 4.63-4.73 (1H, m, CHBnz) 7.19-7.34 (5H, m, C₆H₅)

### Example 1b

### N(4-(t-Butoxy)-2R-isobutylsuccinyl)-4S-phenylmethyl-2-oxazolidinone

N-(4-Methylpentanoyl)-4S-phenylmethyl-2-oxazolidinone (20g, 0.0726 mol) was placed in a dry 1 litre 3-necked flask to which was added dry THF (400 ml). The mixture was kept under a stream of Argon and cooled to -78°C (dry ice/acetone). Sodium hexamethyldisilylamide (1M solution in THF, 0.0726 mol, 72.6 ml) was added dropwise through a dropping funnel (it was added to the funnel via syringe). After stirring for 20 minutes, t-butylbromoacetate (21.02g, 15.8 ml, 0.1089 mol, 1.5 equiv.) was added dropwise over 1 minute, to give an orange solution. The mixture was kept at -78°C and allowed to warm to -50°C over 2 hours (after which time it turned pink). The reaction was then quenched by adding acetic acid (10.90g, 10.4 ml, 0.1815 mol, 2.5 equiv.) in ether (50 ml) at -50°C whereupon the solution became colourless. The solvent was removed and the resulting slurry partitioned between ethyl acetate and brine. The ethyl acetate layer was washed once with brine and the original brine layer was back-extracted with ethyl acetate. The combined organic layers were dried and the solvent removed, giving a yellow oil which crystallized on cooling overnight to yield the title compound as a crystalline solid (21.36g, 76%).

Analysis calculated for C₂₂H₃₁O₅N MWt = 389.48
delta_{H} (250 MHz, CDCl₃) 0.91-0.96 (6H, dd, CMe₂, J=4.5 Hz), 1.44 (9H, s, CMe₃) 1.24-1.72 (3H, m, CH₂CHMe₂), 2.49 (1H, dd, CH₂Ph, J=4.6 Hz), 2.72 (1H, dd, CH₂CO₂CH(CH₃)₃, J=2.3 Hz), 3.36 (1H, dd, CH₂Ph, J=3.25 Hz), 4.16-4.18 (2H, m, CH2OCO), 4.20-4.35 (1H, m, CH-CO), 4.62-4.72 (1H, m, CHBz), 7.24-7.38 (5H, m, C₆H₅)
[alpha]²⁵D = + 66.9 (c=1, MeOH)

### Example 1c

### 4-(t-Butoxy)-2R-isobutylsuccinic acid

N(4-(t-Butoxy)-2R-isobutylsuccinyl)-4S-phenylmethyl-2-oxazolidinone (15.30g, 0.039 mol) was placed in a 1 litre flask with a stirrer bar and to it was added 750 ml of 4.1 THF:H₂0. This solution was stirred and cooled to 0°C (ice/acetone bath) then 60% aq. H₂O₂ (4.5 ml, 0.157 mol, 4 equiv) was added via syringe over 5 mins, followed by Li(OH)₂ (2.65g, 0.063 mol, 1.6 equiv.) in 100 ml water. The reaction mixture was stirred for 1h at 0°C. TLC (10% methanol/dichloromethane) showed complete reaction (product gave a yellow spot on TLC on staining with bromocresol green and heating). The reaction mixture was quenched with NaNO₂ (10.88g, 0.157 mol, 4 equiv.), the final pH was 12-13. THF was removed in-vacuo and the aqueous layer extracted with dichloromethane (3 x 200 ml) to recover the chiral auxliary. The organic extracts were dried (MgSO₄ anhyd.), solvent removed in-vacuo and the resulting solid chiral auxiliary (7.05g, 0.039 mol, 100%) recrystallised from ethyl acetate/hexane (2:1)
[alpha]²⁵D = 13.0° (x=1, MeOH)
[alpha]²⁵D 4.9° (c=1, EtOH)
The aqueous layer was cooled in an ice bath and acidified to pH 5-6 with 2M HCl. The resulting cloudy solution was extracted with ethyl acetate (4 x 200 ml), readjusting the pH to 5-6 in between extractions. The combined organic extracts were dried over MgSO₄, filtered and the solvent was removed to yield the title compound as a pale yellow oil (8.21g, 91%).
delta_{H} (250 MHz, CDCl₃) 0.93 (6H, dd, J=7, 8Hz), 1.28 (1H, m), 1.64 (1H, m), 2.38 (1H, dd, J=16, 5Hz), 2.59 (1H, dd, J=16, 9Hz), 2.85 (1H, m).
[alpha]²⁵D = + 10.4 (c=1, MeOH)

### Example 1d

### Pentafluorophenyl 4-(t-butoxy)-2R-isobutylsuccinate

A solution of the chiral acid (from example 1c, 5.0g, 21.7 mmol) and pentafluorophenol (8.0g, 43 mmol) in dichloromethane (50 ml) was cooled to 0°C before dropwise addition of N-methylmorpholine (2.7g, 26.7 mmol) followed by water-soluble carbodiimide (5.5g, 28.7 mmol) in several portions. After the WSCDI had dissolved, a small amount of white insoluble material remained which did not dissolve on addition of N,N-dimethylformamide (5 ml). The mixture was allowed to warm to room temperature and was then stirred overnight at room temperature.

Solvents were removed on a rotary evaporator and the residue was resuspended in dichloromethane (100 ml) and washed successively with 1M HCl (2 x 200 ml), 0.5M Na₂CO3 (2 x 200 ml) and brine (200 ml) and dried (Na₂SO₄). TLC (CH₂Cl₂) showed a single UV-active spot (R_{f}ca.0.8) with a small amount of brown baseline impurity. The solution was therefore evaporated to a brown oil and flushed through a silica column (2 x 20 cm) with dichloromethane. UV-positive fractions were pooled and evaporated to give the pentafluorophenol ester as a pale yellow oil (8.31g, 97%).
C₁₈H₂₁F₅O₄ MWt = 396.35
i.r. (neat) 1785, 1732 cm⁻¹
delta_{H} (250 MHz, CDCl₃) 3.23 (1H, m), 2.74, 2.52 (2H, ddd, J=9.3, 5.2, 16.8 Hz), 1.75 (2H, m), 1.46 (10H, s and m), 0.98, 0.96 (6H, 2d, J=6.6 Hz)
delta_{C} (250 MHz, CDCl₃) 171.3, 170.3, 143.2-138.9, 81.4, 41.0, 39.5, 37.7, 28.0, 25.8, 22.6, 22.1

### Example 1e

### O-Benzyl-L-tyrosine N-methylamide

N-Boc-O-benzyl tyrosine methylamide (5.29g, 13.8 mmol) was taken up in CH₂Cl₂ (100 ml). To the solution at O°C TFA (10 ml) was added dropwise and the solution allowed to warm to ambient temperature. After 4 hours the solvent and TFA were removed under vacuum. Any remaining TFA was quenched with saturated NaHCO₃ solution (100 ml). The reaction mixture was extracted using CH₂Cl₂ (100 ml) and washed with saturated NaHCO₃ solution (100 ml) and brine (100 ml). The CH₂Cl₂ layer was dried over Na₂SO₄ and the solvent was removed under vacuum to give a white solid, which was recrystallised from ethyl acetate/hexane to yield the title compound as a white crystalline solid (3.35g, 85.4%).
delta_{H} (250 MHz, CDCl₃) 7.45-7.33 (5H, bm, Bn-H), 7.23 (1H, bs, CONHMe), 7.13 (2H, d, J=8.5 Hz, Ar-H), 6.93 (2H, d, J= 8.6 Hz, Ar-H), 5.06 (2H, s, CH₂), 3.56 (1H, dd, J=5.1, 4.0 Hz, CH), 3.20 (1H, dd, J=9.8, 4.0 Hz, H of CH₂), 2.82 (3H, d, J=5.0 Hz, NHCH₃), 2.65 (1H, dd, J=9.3, 4.5 Hz, H of CH₂), 1.35 (2H, bs, NH₂).

### Example 1f

### [4-(t-Butoxy)-2R-isobutylsuccinyl]-L-(4-benzyloxy) phenylalanine-N-methylamide

To a stirred solution of the O-benzyl-L-tyrosine-N-methylamide (from example 1e, 3g, 10.6 mmol) in DMF (100 ml) was added the chiral pentafluorophenyl ester (from example 1d, 8.37g, 21.1 mmol). The resulting solution was stirred at room temperature overnight. The DMF was removed under vacuum. The residue was taken up in CH₂Cl₂ (200 ml) and washed with saturated NaHCO₃ (2 x 100 ml), citric acid (2 x 100 ml) and brine (100 ml). The organic layer was dried over MgSO₄ and the solvent removed under vacuum to give a clear oil. Flash chromatography (flash silica, CH₂Cl₂ to 5% MeOH/CH2Cl2) gave the title compound as a pale yellow solid (4.95g, 94%).
C₂₉H₄₀N₂O₅MWt = 496.65
delta_{H} (250 MHz, CDCl₃) 7.45-7.31 (5H, m, CH-21 to 25), 7.15 (2H, d, J=8.6 Hz, CH-9,11), 6.91 (2H, d, J=8.6 Hz, CH-8,12), 6.30 (1H, d, J=7.8 Hz, CONH), 5.92 (1H, m, CONHMe), 5.04 (2H, s, CH₂-19), 4.50 (1H, q, J=7.8 Hz, CH-5), 3.10 (1H, dd, J=6.3, 6.2 Hz, CH₂-6a), 2.82 (1H, dd, J=7.8 Hz, CH₂-6b), 2.70 (3H, d, J=4.8Hz, CH₃-13), 2.61 (1H, m, CH-3), 2.46 (2H, m, CH₂-2a, 2b), 1.52 (2H, m, 1.44, CH₂-15), (9H, s, CH₃-27, 28, 29), 1.19 (1H, m, CH-16), 0.88, 0.85 (6H, 2d, J=6.5, 6.3 Hz, CH₃-17, 18).

### Example 1g

### [4-(t-Butoxy)-2R-isobutylsuccinyl]-L-(4-hydroxy)-phenylalanine-N-methylamide

[4-(t-Butoxy)-2R-isobutylsuccinyl)-L-(4-benzyloxy) phenylalanine-N-methylamide (2.19g, 4.4 mmol) was taken up in 10% cyclohexene/ethanol (30 ml) and 10% Pd/charcoal (0.219g) added. The mixture was then heated under reflux. After 3 hours the hot solution was filtered through glass fibre paper and the black solid washed with methanol. The filtrate was concentrated under reduced pressure to give the title compound as a white foam (1.78g, 99%).
C₂₂H₃₄N₂O₅ MWt = 405.6
delta_{H} (250 MHz, CDCl₃) 7.08 (2H, d, J=8.6 Hz, CH-9, 11), 6.76 (2H, d, J=8.6 Hz, CH-8, 12), 6.35 (1H, d, J=8.0 Hz, CONH), 5.91 (1H, m, CONHMe), 4.50 (1H, q, J=7.9 Hz, CH-5), 3.06 (1H, dd, J=6.2Hz, CH₂-6a), 2.96 (1H, dd, J=7.9 Hz, CH₂-6b), 2.71 (3H, d, J=4.8 Hz, CH₃-13), 2.61 (1H, m, CH-3), 2.48 (2H, m, CH₂-2a, 2b), 1.52 (2H, m, CH₂-15), 1.44 (9H, s, CH₃-20, 21, 22), 1.25 (1H, m, CH-16), 0.86 (6H, d, J=6.4 Hz, CH₃-17, 18).
delta_{C} (250 MHz, CDCl₃) 173.8, 170.4, 154.2, 128.8, 126.3, 114.2, 79.8, 76.1-75.1, 53.7, 39.6, 36.7, 36.1, 26.6, 24.8, 24.2, 21.2, 20.9.

### Example 1h

### [4-(t-Butoxy)-2R-isobutylsuccinyl]-L-(4-oxymethylcarboxybenzyl)-phenylalanine-N-methylamide

[4-(t-Butoxy)-2R-isobutylsuccinyl)-L-(4-hydroxy) phenylalanine-N-methylamide (2.69g, 6.6 mmol) wan taken up in dry acetone (150 ml). Anhydrous Na₂CO₃ (0.84g, 7.9 mmol) was added with stirring, followed by dropwise addition of benzyl-2-bromoacetate (2.27g, 9.9 mmol). The reaction flask was flushed with argon and then the reaction mixture heated under reflux. After 48 hours the solvent was removed under vacuum. The residue was taken up in CH₂Cl₂ (100 ml) washed with saturated Na₂CO₃ (100 ml), 1M HCl (100 ml) and brine (100 ml), dried over MgSO₄ and the CH₂Cl₂ removed under vacuum to give a yellow oil. Flash chromatography (flash silica, 2% MeOH/CH₂Cl₂) gave the title compound as a white solid (1.91g, 52%).
C₃₁H₄₂N₂O₇ MWt = 554.69
delta_{H} (250 MHz, CDCl₃) 7.36 (5H, s, CH-23-27), 7.14 (2H, d, J=8.7 Hz, CH-9, 11), 6.83 (2H, d, J=8.7 Hz, CH-8, 12), 6.33 (1H, d, J=7.9 Hz, CONH), 5.92 (1H, m, CoNHMe), 5.24 (2H, s, CH₂-21), 4.64 (2H, s, CH₂-19), 4.48 (1H, m, CH-5), 3.08 (1H, dd, J=6.2 Hz, CH₂-6a), 2.96 (1H, dd, J=7.9 Hz, CH₂-6b), 2.68 (3H, d, J=4.8 Hz, CH₃-13), 2.63 (1H, m, CH-3, 2.46 (2H, m, CH₂-2a,2b), 1.48 (2H, m, CH₂-15), 1.43 (9H, s, CH₃-29, 30, 31), 0.87, 0.84 (6H, 2d, J=6.5 Hz, CH₃-17,18).

### Example 1i

### [4-Hydroxy-2R-isobutylsucccinyl]-L-(4-oxymethylcarboxybenzyl)-phenylalanine-N-methylamide

[4-(t-Butoxy)-2R-isobutylsuccinyl]-L-(4-oxymethylcarboxybenzyl)-phenylalanine-N-methylamide (2.12g, 3.8 mmol) was taken up in 95% TFA/H₂O (50 ml). The solution was stirred at O°C for 3 hours. TFA/H₂O removed under vacuum. The residue was taken up in CH₂Cl₂ (50 ml) washed with brine (3 x 50 ml) dried over MgSO₄ and the solvent removed under vacuum to give the title compound as a white solid (1.89g, 99%).

### Example 1j

### [4-(N-benzyloxyamino)-2R-isobutylsuccinyl]-L-(4-oxymethylcarboxybenzyl)-phenylalanine-N-methylamide

[4-Hydroxy-2R-isobutylsuccinyl]-L-(4-oxymethylcarboxy-benzyl)-phenylalanine-N-methylamide (1.89g, 3.79 mmol) was dissolved in CH₂Cl₂ (20 ml). To the solution was added HOBT (0.63g, 4.17 mmol), WSCDI (1.09g, 5.6 mmol), NMM (0.58g, 5.6 mmol) and after 15 minutes benzylhydroxylamine (0.51g, 4.17 mmol). The reaction mixture was stirred at room temperature. After 16 hours the solvent was removed. The yellow residue was taken up in ethyl acetate whereupon white crystals precipitated out, which were collected by filtration and washed with ethyl acetate to yield the title compound as a white solid (0.58g, 27%).
C₃₄H₄₁N₃O₇ MWt = 603.72
delta_{H} (250 MHz, CDCl₃) 8.59, (1H, m, CoNHOBz), 7.37 (10H, s, CH-23-27, CH-30 to 34), 7.11 (2H, d, J=8.6 Hz, CH-9, 11), 6.80 (2H, d, J=8.6 Hz, CH-8,12), 6.47 (1H, m, CONH), 5.95 (1H, m, CONHMe), 5.21 (2H, s, CH₂-21), 4.87 (2H, m, CH₂-28), 4.63 (2H, s, CH₂-19), 4.52 (1H, m, CH-5), 3.02 (2H, m, CH₂-6a, 6b), 2.71 (3H, d+m, J=4,8 Hz, CH₃-13, CH-3), 2.42 (2H, m, CH₂-2a, 2b), 1.46 (2H, m, CH2-15), 1.18 (1H, m, CH-16), 0.87, 0.84 (6H, 2d, J=6.8, 6.6 Hz, CH₃-17,18).

### Example 1k

### [4-(N-Hydroxyamino)-2R-isobutylsuccinyl]-L-(4-oxymethylcarboxylic acid)-phenylalanine-N-methylamide

[4-(N-benzyloxyamino)-2R-isobutylsuccinyl]-L-(4-oxymethylcarboxybenzyl)-phenylalanine-N-methylamide (467 mg, 0.77 mmol) was taken up in 10% cyclohexene/ethanol (40 ml) and 20% Pd/charcoal (93 mg) added with stirring. The solution was heated under reflux and after 3 1/2 hours the solution filtered through glass fibre paper. The filtrate was concentrated down under reduced pressure to give the title compound as a white solid (322 mg, 98%).
mpt = 168°C

| Analysis calculated for C₂₀H₂₉N₃O₇ MWt= 423.7 | | | |
|---|---|---|---|
| Requires | C 56.73 | H 6.90 | N 9.92 |
| Found | C 56.52 | H 6.91 | N 9.59 |

delta_{H} (250 MHz, MeOD) 7.89 (1H, bd, CONHMe), 7.11 (2H, d, J=8.4 Hz, CH-9,11), 6.81 (2H, d, J=8.4 Hz, CH-8.12), 4.56 (2H, s, CH₂-19), 4.44 (1H, m, CH-5), 3.05 (1H, dd, J=6.4, 6.3 Hz, CH₂-6a), 2.87 (1H, dd, J=8.8, 9.0 Hz, CH₂-6b), 2.64 (3H, s,CH₃-13), 2.78-2.58 (1H, bm, CH-3), 2.16 (1H, dd, J=4.7, 7.8 Hz, CH-2a), 2.04 (1H, dd, J=6.5, 6.6 Hz, CH-2b), 1.36 (2H, m, CH2-15), 1.13 (1H, m, CH-16), 0.81, 0.77 (6H, 2d, J=6.3 Hz, CH₃-17, 18).
delta_{C} (250 MHz, DMSO) 173.34, 170.93, 169.80, 167.17, 155.8, 130.2, 129.5, 113.5, 64.1, 53.7, 40.4, 40.2, 40.0, 38.03, 35.86, 35.16, 25.10, 24.63, 22.78, 21.41.

### Example 2

### [4-(N-Hydroxyamino)-2R-isobutylsuccinyl]-L-(4-oxymethylcarboxy-N-methylamide)phenylalanine-N-methylamide

### Example 2a

### [4-(t-Butoxy)-2R-isobutylsuccinyl]-L-(4-oxymethylcarboxylic acid)-phenylalanine-N-methylamide

Utilising the procedure described in example 1g but employing [4-(t-Butoxy)-2R-isobutylsuccinyl]-L-(4-oxymethylcarboxybenzyl)-phenylalanine-N-methylamide (from example 1h, 9.90 g, 17.85 mmol) in lieu of [4-(t-Butoxy)-2R-isobutylsuccinyl]-L-(4-benzyloxy)-phenylalanine-N-methylamide yielded, the title compound as a white solid (8.07 g, 97.3%)
delta_{H} (250 MHz, CDCl₃) 7.07 (2H, d, J=8.6 Hz, CH-9, 11), 7.03 (1H, m, CONHCH), 6.79 (2H, d, J=8.6 Hz, CH-8,12), 6.70 (1H, m, CONHMe), 4.63 (1H, m, CH-5), 4.60 (2H, s, CH2-19), 2.96 (2H,d, J=7.1 Hz, CH₂-6), 2.66 (3H, d, J=4.8 Hz, CH₃-13), 2.65 (1H, s, CH-3), 2.65 (1H, s, CH-3), 2.43 (1H, dd, J=8.5 Hz, CH₂-2a), 2.33 (1H, dd, J=5.3 Hz, CH2-2b), 1.45 (2H, m, CH₂-15), 1.41 (9H, s, CH3-21,22,23), 1.20 (1H, m, CH-16), 0.82 (6H, dd, J=6.3, 6.2 Hz, CH₃-17,18).
delta_{C} (250 MHz, CDCl₃) 174.1, 170.7, 170.2, 155.3,
128.9, 128.4, 113.3, 79.6, 76.1-75.1, 63.7, 53.4, 39.8, 39.6, 36.7, 35.8, 26.6, 24.8, 24.2, 21.3, 20.8.

### Example 2b

### [4-(t-Butoxy)-2R-isobutylsuccinyl]-L-(4-oxymethylcarboxy-N-methylamide)-phenylalanine-N-methylamide

[4-(t-Butoxy)-2R-isobutylsuccinyl]-L-(4-oxymethylcarboxylic acid)-phenylalanine-N-methylamide (0.5g, 1.07 mmol) was dissolved in CH₂Cl₂ (100 ml). At 0°C pentafluorophenol (0.4g, 2.15 mmol), WSCDI (0.26g, 1.3 mmol) and N-methylmorpholine (0.11g, 1.1 mmol) were added. After 15 minutes 8M methylamine in ethanol (0.25g, 2.7 mmol) was added dropwise. The solution was allowed to warm to ambient temperature and stirred for 12 hours. A white solid of MeNH₂.HCl precipitated out, but was not collected. The reaction solution was washed with 1M HCl (100 ml), 1M Na₂CO₃ (100 ml) and brine (100 ml). The CH₂Cl₂ layer was dried over MgSO₄ and the solvent removed under reduced pressure to give the title compound as a white solid (0.44g, 86%).
delta_{H} (250 MHz, CDCl3) 7.19 (2H, d, J=8.6 Hz, CH-9, 11), 6.84 (2H, d, J=8.6 Hz, CH-8,12), 6.60 (1H, m, CONHMe), 6.27 (1H, d,J=7.8 Hz, CONH), 5.99 (1H, m, CONHMe), 4.52 to 4.46 (3H, s+q, CH-5, CH₂-19), 3.09 (2H, m, CH₂-6a,6b), 2.92 (3H, d, J=4.8 Hz, CH3-21), 2.72 (3H, d, J=4.8 Hz, CH₃-13), 2.61 (1H, m, CH-3), 2.46 (2H, m, CH₂-2a, 2b), 1.45 (11H, s+m, CH₃-23, 24, 25, CH₂-15) 1.20 (1H, m, CH-16), 0.87, 0.84 (6H, 2d, J=6.3 Hz, CH₃-17,18).

### Example 2c

### [4-(N-Hydroxyamino)-2R-isobutylsuccinyl]-L-(4-oxymethylcarboxy-N-methylamide)phenylalanine-N-methylamide

The title compound was prepared from [4-(t-Butoxy)-2R-isobutylsuccinyl]-L-(4-oxymethylcarboxy-N-methylamide)-phenylalanine-N-methylamide utilising the method described in examples 1i to 1k
mpt = 211°C

| Analysis calculated for C₂₁H₃₂N₄0₆ MWt = 436.51 | | | |
|---|---|---|---|
| Requires | C 57.78 | H 7.39 | N 12.84 |
| Found | C 57.30 | H 7.27 | N 12.56 |

delta_{H} (250 MHz, DMSO) 10.39 (1H, s, CONHOH), 8.74 (1H, s, CONHOH), 7.98 (2H, m, CONHMe), 7.85 (1H, d, J=4.7 Hz, CONH), 7.12 (2H, d, J=8.5 Hz, CH-9,11), 6.83 (2H, d, J=8.6 Hz, CH-8,12), 4.38 (2H, s, CH-19), 4.32 (1H, m, CH-5), 2.96 (1H, dd, J=5.1, 5.0 Hz, CH₂-6a), 2.74 (1H, dd, J=9.6, 9.7 Hz, CH₂-6b), 2.64 (3H, d, J=4.7 Hz, CH₃-21), 2.60 (1H, m, CH-3), 2.55 (3H, d, J=4.5 Hz, CH₃-13), 2.05 (1H, dd, J=3.7, 7.0 Hz CH₂-2a), 1.91 (1H, dd, J=7.5, 7.6 Hz, CH₂-2b), 1.28 (2H, m CH2-15), 0.98 (1H, m, CH-16), 0.77, 0.72 (6H, 2d, J=6.3 Hz, CH₃-17,18)
delta_{C} (250 MHz, DMSO) 173.3, 170.9, 167.6, 167.2, 155.7, 130.5, 129.6, 113.6, 66.7, 53.7, 40.4-36.0, 35.7, 35.2, 25.1, 24.9, 24.6, 22.8, 21.4

### Example 3

### 4-(N-Hydroxyamino)-2R-isobutylsuccinyl]-L-(4-oxymethyl carboxymethyl)-phenylalanine-N-methylamide

### Example 3a

### 4-(t-Butoxy)-2R-isobutylsuccinyl]-L-(4-oxymethyl carboxymethyl)-phenylalanine-N-methylamide

[4-(t-Butoxy)-2R-isobutylsuccinyl]-L-(4-oxymethylcarboxylic acid) phenylalanine-N-methylamide (from example 2a, 0.5g, 1.08 mmol) was dissolved in CH₂Cl₂ (20 ml) and cooled to 0°C. A solution of diazomethane in ether (3 ml) was added via a pipette until gas evolution ceased and the reaction solution remained a pale yellow colour. After 30 minutes the reaction mixture was treated with 10% acetic acid/ether until the solution was colourless and washed with brine (30 ml). The CH₂Cl₂ layer was dried and the solvent removed under reduced pressure to give the title compound as a white solid (0.45g, 87%).
C₂₅H₃₃N₂O₇ MWt = 478.69
delta_{H} (250 MHz, CDCl₃) 7.16 (2H, d, J=8.6 Hz, CH-9, 11), 6.83 (2H, d, J=8.6 Hz, CH-8,12), 6.35 (1H, d, J=8.0 Hz, CONH), 5.91 (1H, m CONHMe), 4.61 (2H, s, CH2-19), 4.49 (1H, q, J=7.9 Hz, CH-5), 3.61 (3H, s, CH3-21), 3.06 (1H, dd, J=6.2 Hz, CH2-6a), 2.96, (1H, dd, J=9.9 Hz, CH2-6b), 2.71 (3H, d, J=4.8 Hz, CH₃-13), 2.59 (1H, m, CH-3), 2.48 (2H, m, CH₂-2a,2b), 1.53 (2H, m, CH₂-15), 1.44 (9H, s, CH₃-23,24,25), 1.25 (1H, m, CH-16), 0.86 (6H, 2d, J=6.4 Hz, CH₃-17,18).

### Example 3b

### 4-(N-Hydroxyamino)-2R-isobutylsuccinyl]-L-(4-oxymethyl carboxymethyl) phenylalanine-N-methylamide

The title compound was prepared from 4-(t-Butoxy)-2R-isobutylsuccinyl]-L-(4-oxymethylcarboxymethyl) phenylalanine-N-methylamide utilising the method described in examples 1i and 1k.
mpt = 187°C

| Analysis calculated for C₂₁H₃₁N₃O₇ MWt = 437.5 | | | |
|---|---|---|---|
| Requires | C 57.65 | H 7.14 | N 9.60 |
| Found | C 57.63 | N 7.11 | N 9.27 |

delta_{H} (250 MHz, DMSO) 10.40 (1H, s, CONHOH), 8.76 (1H, s, CONHOH), 7.99 (1H, d, J=8.0 Hz, CONH), 7.86 (1H, m, CONHMe), 7.12 (2H, d, J=8.4 Hz, CH-9,11), 6.81 (2H, d, J=8.4 Hz, CH-8,12), 4.73 (2H, s, CH₂-19), 4.33 (1H, m, CH-5), 3.69 (3H, s, CH₃-21), 2.97 (1H, dd, J=4.6 Hz, CH₂-6a), 2.76 (1H, dd, J=9.9, 10.1 Hz, CH₂-6b) 2.57, 3H, d, J=4.2 Hz, CH₃-13), 2.62-2.56 (1H, m, CH-3), 2.07 (1H, dd, J=6.9, 6.8 Hz, CH₂-2a), 1.93 (1H, dd, J=7.4, 7.6 Hz, CH₂-2b), 1.29 (2H, m, CH₂-15), 1.03 (1H, m, CH-16), 0.78, 0.74 (6H, 2d, J=6.2 Hz, CH₃-17,18).
delta_{C} (250 MHz, DMSO) 173.3, 170.9, 166.6, 167.2, 155.6, 130.5, 129.6, 113.6, 64.2, 53.7, 51.3, 40.3-38.1, 35.8, 35.1,25.1, 24.6, 22.7, 21.4

### Example 4

### [4-(N-Hydroxyamino)-2R-isobutylsuccinyl]-L-(4-oxymethylcarboxy-N-benzylamide)-phenylalanine-N-methylamide

### Example 4a

### [4-(t-Butoxy)-2R-isobutylsuccinyl]-L-(4-oxymethylcarboxy-N-benzylamide)-phenylalanine-N-methylamide.

Utilising the procedure described in example 2b employing benzylamine (0.25g, 2.4 mmol) in lieu of methylamine yielded the title compound as a white solid (0.53g, 81%).
C₂₉H₄₁N₃O₆ MWt=527
delta_{H} (250 MHz, CDCl₃ 7.40-7.27 (5H, m, CH-23 to 27), 7.17 (2H, d, J=8.6 Hz, CH-9,11), 6.92 (1H, m, CONHBz), 6.85 (2H, d, J=8.6 Hz, CH-8,12), 6.29 (1H, d, J=7.8 Hz, CONH), 5.96 (1H, m, CONHMe), 4.56-4.45 (5H, m, CH-5, CH₂-21, CH₂-19), 3.04 (2H, m, CH₂-6), 2.70 (3H, d, J=4.8 Hz, CH₃-13), 2.58 (1H, m, CH-3), 2.43 (1H, dd, J=8.5 Hz, CH₂-2a), 2.33 (1H, dd, J=5.3 Hz, CH₂-2b), 1.45 (2H, m, CH₂-15), 1.41 (9H, s, CH₃-23,24,25), 1.20 (1H, m, CH-16), 0.86 (6H, 2d, J=6.3, 6.2 Hz, CH₃-17,18).

### Example 4b

### [4-(N-Hydroxyamino)-2R-isobutylsuccinyl]-L-(4-oxymethylcarboxy-N-benzylamide)-phenylalanine-N-methylamide

The title compound was prepared from [4-(t-Butoxy)-2R-isobutylsuccinyl]-L-(4-oxymethylenecarboxy-N-benzylamide) phenylalanine-N-methylamide utilising the method described in examples 1i to 1k.
mpt= 206°C

| Analysis calculated for C₂₇H₃₆N₄O₆ MWt = 512.6 | | | |
|---|---|---|---|
| Requires | C 63.26 | H 7.08 | N 10.93 |
| Found | C 62.52 | H 7.09 | N 10.97 |

delta_{H} (250 MHz, DMSO) 10.41 (1H, s, CONHOH), 8.76 (1H, s, CONHOH), 8.62 (1H, t, J=6.1 Hz, CONHCHPh), 8.01 (1H, d, J=8.3 Hz, CONH), 7.87 (1H, m, CONHMe), 7.26 (5H, m, CH-23 to 27), 7.13 (2H, d, J=8.5 Hz, CH-9,11), 6.86 (2H, d, J=8.5 Hz, CH-8,12), 4.48 (2H, s, CH₂-19), 4.34 (2H, d, J=6.0 Hz, CH₂-21), 2.97 (1H, dd, J=4.5, 4.8 Hz, CH₂-6a), 2.76 (1H, dd, J=9.5, 9.6 Hz, CH₂-6b), 2.64-2.55 (1H, bm, CH-3), 2.56 (3H, d, J=4.4 Hz, CH₃-13) 2.06 (1H, dd, J=3.2, 7.1 Hz, CH₂-2a), 1.92 (1H, dd, J=7.6 Hz, CH₂-2b), 1.28 (2H, m, CH₂-15), 0.97 (1H, m, CH-16), 0.78, 0.73 (6H, 2d, J=6.3 Hz, CH₃-17,18).
delta_{C} (250 MHz, DMSO) 173.5, 170.9, 167.3, 167.2, 155.7, 138.6, 130.5, 129.6, 127.8, 126.8, 126.3, 113.9, 66.6, 53.7, 41.32, 40.1-38.1, 35.2, 25.1, 24.7, 22.8, 21.4

### Example 5

### [4-(N-Hydroxyamino)-2R-isobutylsuccinyl]-L-(4-oxymethylcarboxy-beta-alanine)phenylalanine-N-methylamide

### Example 5a

### [4-(t-Butoxy)-2R-isobutylsuccinyl]-L-(4-oxymethylcarboxy-beta-alanine benzyl ester) phenylalanine-N-methylamide

Utilizing the procedure described in example 2b employing beta-alanine benzyl ester (0.76g, 2.16 mmol) in lieu of methylamine yielded the title compound as a yellow oil (0.65g, 97%).
C₃₄H₄₇N₃0₈ MWt = 625
delta_{H} (250 MHz, CDCl₃) 7.35 (5H, s, CH-26 to 30), 7.17 (2H, d, J=8.5 Hz, CH-9,11), 7.19 (1H, m, CONHCH₂), 6.82 (2H,d, J=8.5 Hz, CH-8,12), 6.33 (1H, d, J=7.8 Hz, CONH), 6.01 (1H, m, CONHMe), 5.13 (2H, s, CH₂-24), 4.49 (1H, q, J=6.7, 7.6 Hz, CH-5), 4.44 (2H, s, CH₂-19), 3.64 (2H, q, J=6.1 Hz, CH₂-21), 3.06 (2H, m, CH₂-6a, 6b), 2.72 (3H, d, J=4.8 Hz, CH₃-13), 2.66-2.62 (3H, m, CH2-22, CH-3), 2.50-2.39 (2H, m, CH₂2a,b), 1.48 (2H, m, CH₂-15), 1.22, (1H, m, CH-16). 0.87, 0.84 (6H, 2d, J=6.4, 6.3 Hz, CH₃-17, 18).

### Example 5b

### [4-(N-Hydroxyamino)-2R-isobutylsuccinyl]-L-(4-oxymethylcarboxy-beta-alanine)phenylalanine-N-methylamide

The title compound was prepared from [4-(t-butoxy)-2R-isobutylsuccinyl]-L-(4-oxymethylcarboxy-beta-alanine benzyl ester)phenyl alanine-N-methylamide utilizing the method described in examples 1i to 1k.
mpt = 195°C

| Analysis calculated for C₂₃H₃₄N₄0₈ MWt = 494.6 | | | |
|---|---|---|---|
| Requires | C 55.86 | H 6.93 | N 11.33 |
| Found | C 55.94 | H 6.96 | N 11.51 |

delta_{H} (250 MHz, MeOD) 7.13 (2H, d, J=8.5 Hz, CH-9,11), 6.86 (2H, d, J=8.5 Hz, CH-8,12), 4.47-4.42 (1H, m, CH-5), 4.42 (2H, s, CH₂-19), 3.48 (2H, t, J=6.7 Hz, CH₂-21), 3.06 (1H, dd, J=6.4, 6.3 Hz, CH₂-6a), 2.85 (1H, dd, J=8.7, 8.8 Hz, CH₂-6b), 2.80-2.64 (1H, m, CH-3), 2.64 (3H, s, CH₃-13), 2.50 (2H, t, J=6.7 Hz, CH₂-22), 2.17 (1H, dd, J=6.5 Hz, CH₂-2a), 2.04 (1H, dd, J=6.5 Hz, CH₂-2b) 1.36 (2H, m, CH₂-15), 1.06 (1H, m, CH-16), 0.81, 0.77 (6H, 2d, J=6.3 Hz, CH₃-17,18).
delta_{C} (250 MHz, MeOD) 177.0, 175.5, 173.9, 171.1, 170.6, 157.9, 132.0, 131.4, 115.8, 68.3, 56.2, 50.0, 49.6-47.9, 42.5, 42.4, 37.9, 36.8, 36.0, 34.6, 26.8, 26.3, 23.5, 22.

### Example 6

### [4-(N-Hydroxyamino)-2R-isobutylsuccinyl]-L-(4-oxymethyl carboxy-glycine methyl ester) phenylalanine-N-methylamide

### Example 6a

### [4-(t-Butoxy)-2R-isobutylsuccinyl]-L-4-oxymethyl carboxy-glycine methylester)-phenylalanine-N-methylamide.

Utilizing the procedure described in example 2b employing glycine methyl ester hydrochloride (0.13g, 1.07 mmol) in lieu of methylamine yielded the title compound as a white solid (0.45g, 78.5%).
C₂₇H₄₁N₈0₈ MWt = 535.64
delta_{H} (250 MHz, CDCl₃) 7.17 (2H, d, J=8.6 Hz, CH-9,11), 7.15 (1H, m, CH₂CONHCH₂), 6.83 (2H, d, J=8.6 Hz, CH-8), 6.45 (1H, d, J=8.0 Hz, CHCONHCH), 6.26 (1H, m, CONHMe), 4.53 (1H, m, CH-5), 4.48 (2H, s, CH₂-19), 4.13 (2H, d, J=6.3 Hz, CH₂-21), 3.76 (3H, s, CH₃-23), 3.04 (2H, m, CH₂-6), 2.72 (3H, d, J=4.8 Hz, CH₃-13), 2.51 (1H, m, CH-3), 2.42 (2H, m. CH₂-2), 1.47 (2H, m, CH₂-15), 1.43 (9H, s, CH₃-24,25,26), 1.19 (1H, m, CH-16), 0.83 (6H, dd, J=6.3 Hz, CH3-17,18).

### Example 6b

### [4-(N-Hydroxyamino)-2R-isobutylsuccinyl]-L-(4-oxymethyl carboxy-glycinemethylester)-phenylalanine-N-methylamide

The title compound was prepared from [4-(t-Butoxy)-2R-isobutylsuccinyl]-L-4-oxymethyl carboxy-glycine methyl ester)-phenylalanine-N-methylamide utilizing the method described in examples 1i to 1k.
mpt = 180-185°C

| Analysis calculated for C₂₃H₃₄N₄0₈ MWt = 494.55 | | | |
|---|---|---|---|
| Requires | C 55.86 | H 6.93 | N 11.33 |
| Found | C 53.45 | H 6.83 | N 11.50 |

delta_{H} (250 MHz, MeOD) 7.14 (2H, d, J 8.6 Hz, CH-9,11)6.89, (2H, d, J=8.6 Hz, CH-8,12), 4.49 (2H,s, CH₂-19), 4.44 (1H, m, CH-5), 3.99 (2H, s, CH₂-21), 3.69 (3H, s, CH₃-23), 3.11-2.81 (2H, m, CH₂-6), 2.72-2.63 (1H, m, CH-3), 2.64 (3H, s, CH₃-13), 2.21 (1H,dd, J=7.8 Hz, CH₂-2a), 2.04 (1H, dd, J=6.7,6.6 Hz, CH₂-2b), 1.35 (2H, m, CH₂-15) 1.05 (1H, m, CH-16), 0.79 (6H, dd, J=6.4 Hz, CH₃-17,18).
delta_{C} (250 MHz, MeOD) 177.1, 173.9, 171.8, 171.6, 170.6, 158.0, 132.1, 131.4, 115.9, 68.3, 56.3, 52.7, 50.0-48.0, 42.6, 42.4, 41.5, 37.9, 36.8, 26.8, 26.3, 22.3

### Example 7

### [4-(N-Hydroxyamino)-2R-isobutylsuccinyl]-L-(4-oxymethyl carboxyethyl)-phenylalanine-N-methylamide.

### Example 7a

### [4-(t-Butoxy)-2R-isobutylsuccinyl]-L-(4-oxymethyl carboxyethyl)-phenylalanine-N-methylamide.

When the procedure described in example 1g was utilized employing [4-(t-butoxy)-2R-isobutylsuccinyl]-L-(4-oxymethylcarboxybenzyl ester) phenylalanine-N-methylamide (from example 1h 9.10g, 16.41 mmol) in lieu of [4-(t-Butoxy)-2R-isobutylsuccinyl]-L-(4-benzyloxy) phenylalanine-N-methylamide a transesterification reaction occurred and an ethyl group was transferred from the solvent to the reactant molecule. The reaction yielded after chromatography (flash silica, 100% ethyl acetate) the title compound as a white solid (0.51g, 6.3%)
C₂₆H₄₆N₂O₇ MWt = 492.62
¹Hnmr delta_{H} (250 MHz, CDCl₃) 7.09 (2H, d, J=8.6 Hz, CH-9,11), 6.77 (2H, d, J=8.6 Hz, CH-8,12), 6.68 (1H, d, J=8.8 Hz, CONHCH), 6.57 (1H, m, CONHMe), 4.55 (1H, m, CH-5), 4.53 (2H, s, CH₂-19), 4.21 (2H, q, J=7.1 Hz, CH₂-21), 2.97 (2H, m, CH₂-6), 2.72-2.52 (1H, m, CH-3), 2.65 (3H, d, J=4.8 Hz, CH₃-13), 2.43 (1H, dd, J=7.8 Hz, CH₂-2a), 2.27 (1H, dd, J=6.8 Hz, CH₂-2b), 1.42 (2H, m, CH₂-15), 1.37 (9H, s, CH₃-23,24,25), 1.25 (3H, t, J=7.1 Hz, CH₃-22), 1.14 (1H, m, CH-16), 0.78 (6H, dd, J=6.4 Hz, CH₃-17,18).

### Example 7b

### [4-(N-Hydroxyamino)-2R-isobutylsuccinyl]-L-(4-oxymethyl carboxyethyl)-phenylalanine-N-methylamide.

The title compound was prepared from [4-(t-butoxy)-2R-isobutylsuccinyl]-L-(4-oxymethylcarboxyethyl)-phenylalanine-N-methylamide utilising the method described in examples 1i to 1k.
mpt 185°C

| Analysis calculated for C₂₂H₃₃N₃0₇ MWt = 451.52 | | | |
|---|---|---|---|
| Requires | C 58.52 | H 7.37 | N 9.31 |
| Found | C 58.54 | H 7.28 | N 9.26 |

delta_{H} (250 MHz, MeOD) 7.11 (2H, d, J=8.6 Hz, CH-9,11), 6.79 (2H,d, J=8.6 Hz, CH-8,12), 4.61 (2H, s, CH₂-19), 4.43 (1H, m, CH-5), 4.19 (2H, q, J=7.1, 7.2 Hz,CH₂-21), 3.05 (1H, dd, J=6.4, 6.4 Hz, CH₂-6a), 2.81 (1H, dd, J=9.1, 8.9 Hz, CH₂-6b), 2.64 (3H, s, CH₃-13), 2.78-2.58 (1H, m, CH-3), 2.12 (1H, dd, J=7.8, 7.9 Hz, CH₂-2a), 2.06 (1H, dd, J=6.7, 6.8 Hz, CH₂-2b), 1.31 (2H,m, CH₂-15), 1.24 (3H, t, J=7.1 Hz, CH₃-22) 1.09 (1H, m, CH-16), 0.79 (6H, dd, J=6.4 Hz, CH₃-17, 18).
delta_{C} (250 MHz, MeOD) 177.2, 174.0, 171.0, 170.6, 158.1, 131.8, 131.2, 115.6, 66.4, 62.4, 56.4, 50.1,-48.0, 42.6, 42.4, 38.1, 36.9, 26.8, 26.4, 23.6, 22.4, 14.

### Example 8

### [4-(N-Hydroxyamino)-2R-isobutylsuccinyl]-L-(4-oxymethyl carboxyglycine)phenylalanine-N-methylamide

### Example 8a

### [4-(t-Butoxycarbonyl)-2R-isobutylsuccinyl]-L-(4-oxymethylcarboxyglycine benzyl ester)-phenylalanine-N-methylamide

Utilizing the procedure described in example 2b employing glycine benzyl ester hydrochloride (0.24g, 1.21 mmol) in lieu of methylamine yielded the title compound as a white solid (0.55g, 89.5%).
C₃₃H₄₅N₃0₈ MWt = 611.74
delta_{H} (250 MHz, CDCl₃) 7.38 (5H, m, CH-24 to 29), 7.22-7.11 (1H, m, CONHCH₂CO₂Bz), 7.17 (2H, d, J=7.76 Hz, CH-9, 11), 6.84 (2H, d, J=7.6 Hz, CH-8, 12), 6.47 (1H, d, J=8.1 Hz, CHCONHCH), 6.24 (1H, m, CONHMe), 5.19 (2H, s, CH₂-23), 4.62-4.42 (1H, m,CH-5), 4.48 (2H, s, CH₂-19), 4.16 (2H, d, J=6.5 Hz, CH₂-21), 3.05 (2H, m, CH₂-6), 2.77-2.55 (1H, m, CH-3), 2.73 (3H, d, J=4.2 Hz, CH₃-13), 2.53 (1H, dd, J=6.6 Hz, CH₂-2a), 2.35 (1H, dd, J=5.3 Hz, CH₂-2b), 1.55-1.37 (2H, m, CH₂-15), 1.45 (9H, s, CH₃-30,31,32), 1.22, (1H, m, CH-16), 0.97 (6H, m, CH₃-17,18).

### Example 8b

### [4-(N-Hydroxyamino)-2R-isobutylsuccinyl]-L-(4-oxymethyl carboxyglycine)phenylalanine-N-methylamide

The title compound was prepared from [4-(t-butoxycarbonyl)-2R-isobutylsuccinyl]-L-(4-oxymethylcarboxyglycine benzyl ester)-phenylalanine-N-methylamide utilizing the method described in examples 1i to 1k.
mpt = 142.5°C

| Analysis calculated for C₂₂H₃₂N₄0₈ MWt = 480.52 | | | |
|---|---|---|---|
| Requires | C 54.99; | H 6.71 | N 11.66 |
| Found | C 54.19; | H 6.87 | N 10.96 |

delta_{H} (250 MHz, DMSO) 10.42 (1H, s, CONHOH), 8.32 (1H, m, CONHCH₂CO₂H), 8.01 (1H, d, J=8.1 Hz, CHCONHCH), 7.87 (1H, m, CONHMe), 7.13 (2H, d, J=7.6Hz, CH-9,11), 6.87 (2H, d, J=7.3 Hz, CH-8,12), 4.46 (2H, s, CH₂-21), 4.33 (1H, m, CH-5), 3.80 (2H, d, J=5.5 Hz, CH₂-19), 2.96 (1H, dd, J=4.6, 4.5 Hz, CH₂-6a), 2.75 (1H, m, CH₂-6b), 2.56 (3H, s, CH₃-13), 2.68-2.43 (1H, m, CH-3), 2.05 (1H, dd, J=7.0, 6.6 Hz, CH₂-2a), 1.91 (1H, dd, J=5.3 Hz, CH₂-2b), 1.26 (2H, m, CH₂-15), 1.05 (1H, m, CH-16), 0.75 (6H, dd, J=5.7 Hz, CH₃-17,18).
delta_{C} (250 MHz, DMSO) 173.4, 170.9, 170.5, 167.6, 167.2, 155.6, 130.6, 129.6, 113.9, 66.5, 53.7, 40.3, 40.2, 39.7-38.4, 5.9, 35.2, 25.9, 25.1, 22.8, 21.4

### Example 9

### [4-(N-Hydroxyamino)-2R-isobutylsuccinyl]-L-(4-oxymethyl carboxy-N,N-dimethylamide)-phenylalanine-N-methylamide

### Example 9a

### (4-(t-Butoxy)-2R-isobutylsuccinyl]-L-(4-oxymethyl carboxy-N,N-dimethylamide)-phenylalanine-N-methylamide.

Utilising the procedure described in example 2b employing dimethylamine hydrochloride (0.11g, 1.30 mmol) in lieu of methylamine yielded the title compound as a white solid (0.49g, 92.3%).
C₂₆H₄₁N₃0₆ MWt = 491.63
delta_{H} (250 MHz, CDCl₃) 7.13 (2H, d, J=8.5 Hz, CH-9,11), 6.85 (2H, d, J=8.5 Hz, CH-8,12), 6.42 (1H, d, J=8.0 Hz, CHCONHCH), 6.19 (1H, m, CONHMe), 4.63 (2H, s, CH₂-19), 4.50 (1H, m, CH-5), 3.12-2.82 (2H, m, CH₂-6), 3.07 (3H, s, CH₃-21), 2.96 (3H, s, CH₃-22), 2.68 (3H, d, J=4.5 Hz, CH₃-13), 2.62 (1H, m, CH-3), 2.52 (1H, dd, J=8.5 Hz, CH₂-2a), 2.33 (1H, dd, J=4.5 Hz, CH₂-2b), 1.55-1.35 (2H, m, CH₂-15), 1.42 (9H, s, CH₃-23,24,25), 1.19 (1H, m, CH-16), 0.83 (6H, dd, J=6.3 Hz, CH₃-17,18).

### Example 9b

### [4-(N-Hydroxyamino]-2R-isobutylsuccinyl]-L-(4-oxymethyl carboxy-N,N-dimethylamide)-phenylalanine-N-methylamide

The title compound was prepared from [4-(t-Butoxy)-2R-isobutylsuccinyl]-L-(4-oxymethylcarboxy-N,N-dimethylamide)-phenylalanine-N-methylamide utilising the method described in examples 1i to 1k.
mpt 197°C

| Analysis calculated for C₂₂H₃₄N₄0₆ MWt = 450.54 | | | |
|---|---|---|---|
| Requires | C 58.65 | H 7.61 | N 12.44 |
| Found | C 58.58 | H 7.54 | N 12.33 |

delta_{H} (150 MHz, DMSO) 0.42 (1H, s, CONHOH), 8.77 (1H, s, CONHOH), 7.99 (1H, d, J=8.0 Hz, CHCONHCH), 7.87 (1H, m, CONHMe), 7.09 (2H, d, J=8.5 Hz, CH-9,11), 6.78 (2H, d, J=8.5Hz, CH-8,12), 4.71 (2H, s, CH₂-19), 4.32 (1H, m, CH-5), 2.98 (3H, s, CH₃-21), 2.94 (1H, m, CH₂-6a), 2.83 (3H, s, CH₃-22), 2.73 (1H, m, CH₂-6b), 2.55 (3H, m, CH₃-13), 2.68-2.45 (1H, m, CH-3), 2.01 (1H, dd, J=6.8 Hz, CH₂-2a), 1.91 (1H, dd, J=6.8 Hz, CH₂-2b), 1.28 (2H, m, CH₂-15), 0.99 (1H, m, CH-16), 0.74 (6H, dd, J=6.3, 6.2 Hz, CH₃-17,18).
delta_{C} (250 MHz, CDCl₃) 173.4, 170.9, 167.2, 166.8, 156.1, 130.0, 129.4, 113.7, 65.5, 53.7, 40.9-38.0, 35.9, 35.2, 34.5, 25.1, 24.6, 21.4

### Example 10

[4-(N-Hydroxyamino)-2R-isobutylsuccinyl]-L-(4-oxymethylcarboxamide)-phenylalanine-N-methylamide (BB 802)

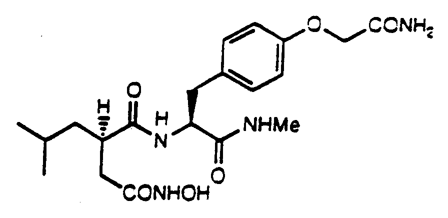

### a) [4-t-Butoxy-2R-isobutylsuccinyl]-L-(4-oxymethylcarboxamide)-phenylalanine-N-methylamide.

[4-t-Butoxy-2R-isobutylsuccinyl]-L-(4-oxymethylcarboxylic acid)-phenylalanine-N-methylamide (see example 2a. 1.10g, 2.36 mmol) was dissolved in DMF (10ml) and cooled to 0°. Pentafluorophenol (0.87g, 4.72 mmol), aqueous ammonia (35%, 0.14g, 2.83 mmol), N-methyl morpholine (0.29g, 2.83 mmol) and WSCDI (0.54g, 2.83 mmol) were added and the reaction stirred overnight. The solvent was removed under high vacuum to leave a yellow oil which was dissolved in DCM and washed sequentially with saturated sodium carbonate, 2M hydrochloric acid and brine. The organic layer was dried over magnesium sulphate, filtered then the sovent removed to leave a white solid (0.89g, 1.92 mmol, 81%): ¹H-NMR; δ (CDCl₃), 7.17 (2H, d, J=8.6 Hz, Aryl-H), 6.83 (2H, d, J=8.6 Hz, Aryl-H), 6.57 (2H, bs, CONH₂), 6.29 (1H, bq, NHMe), 6.17 (1H, bs, CONHCH), 4.57 (1H, q, J=7.0 Hz, COCHNH), 4.44 (2H, s, OCH₂CO), 3.04 (2H, d, J=7.0 Hz, CHCH₂Ar), 2.71 (3H, d, J=4.8 Hz, NHCH₃), 2.60 (1H, m, ⁱBuCH), 2.50 (1H, dd, J= 14, 9 Hz, CH₂CONHOH), 2.33 (1H, dd, J=14, 5 Hz, CH₂CONHOH), 1.43 (11H, m + s, (CH₃)₂CHCH₂ and (CH₃)₃CO), 1.17 (1H, m, (CH₃)₂CHCH₂), 0.86 (3H, d, J=6.4 Hz, CH(CH₃)₂), and 0.83 (3H, d, J=6.3 Hz, CH(CH₃)₂).

### b) [4-(N-Benzylamino)-2R-isobutylsuccinyl]-L-(4-oxymethylcarboxamide)-phenylalanine-N-methylamide

[4-t-Butoxy-2R-isobutylsuccinyl]-L-(4-oxymethylcarboxamide)-phenylalanine-N-methylamide (0.88g, 1.90 mmol) was taken up in trifluoroacetic acid/water (95/5) and stored at 4° overnight. The trifluoroacetic acid was removed by evaporation and the residue taken up in DCM and washed with 2M sodium carbonate solution. The precipitated product was filtered off and dried to give the acid (0.57g, 1.40 mmol) as a white solid.

The acid from above (0.57g, 1.40 mmol) was dissolved in the minimum quantitiy of DMF. Pentafluorophenol (0.52g, 2.80 mmol), O-benzylhydroxylamine (0.34g, 2.80 mmol), N-methylmorpholine (0.18g, 1.82 mmol) and WSCDI (0.35g, 1.82 mmol) were added and the mixture stirred at room temperature overnight. The precipitated product was collected by filtration and washed with cold DCM, giving the title compound as a white solid (0.35g, 0.68 mmol. 49%): ¹H-NMR; δ (DMSO-d₆), 11.05 (1H, s, NHOH), 8.04 (1H, bd, J=8 Hz, CONHCH), 7.86 (1H, bm, NHMe), 7.5 - 7.3 (7H, s + m, CONH₂ + Ph), 7.12 (2H, d, J=8.6 Hz, Aryl-H), 6.82 (2H, d, J=8.6 Hz, Aryl-H), 4.75 (2H, s, COH₂Ph), 4.33 (3H, bs, COCHNH and OCH₂CO), 2.93 (1H, dd, J=14, 5 Hz, CHCH₂Ar), 2.79 (1H, dd, J=14.10 Hz, CHCH₂Ar), 2.60 (1H, m, ⁱBuCH), 2.55 (3H, d, J=4.8 Hz, NHCH₃), 2.08 (1H, dd, J= 14, 7 Hz, CH₂CONHOH), 1.94 (1H, dd, J=14, 7 Hz, CH₂CONHOH), 1.25 (2H, m, (CH₃)₂CHCH₂), 0.95 (1H, m, (CH₃)₂CHCH₂), 0.78 (3H, d, J=6.4 Hz, CH(CH₃)₂), and 0.73 (3H, d, J=6.3 Hz, CH(CH₃)₂).

### c) [4-(N-Hydroxyamino)-2R-isobutylsuccinyl]-L-(4-oxymethylcarboxamide )- phenylalanine-N-methylamide.

[4-(N-Benzylamino)-2R-isobutylsuccinyl]-L-(4-oxymethylcarboxamide)-phenylalanine-N-methylamide (0.34g, 0.66 mmol) was dissolved in 10% cyclohexene/ethanol. 10% palladium on charcoal (40mg) added and the mixture heated at reflux for 1 hour. The reaction mixture was filtered hot then the solvents removed to leave the product as a white solid: m.p. 206.0 - 206.0°: ¹H-NMR; δ (Methanol-d₄), 7.13 (2H, J =8.6 Hz, aryl-H), 6.86 (2H, d, J =8.6 Hz, aryl-H), 4.51-4.38 (1H, m, NCHCO), 4.41 (2H, s, COCH₂CONH₂), 3.06 (1H, dd, J =6.4 Hz, CHCH₂Ph), 2.84 (1H, dd, J=8.9 Hz, CHCH₂Ph), 2.71-2.57 (1H, m, ⁱBuCH), 2.68 (3H, s, CONHCH₃), 2.14 (1H, dd, J= 8, 10 Hz, CHCH₂CONHOH), 2.04 (1H, dd, J=6, 7 Hz, CHCH₂CONHOH), 1.36 (2H, m, (CH₃)₂CHCH₂CH), 1.07 (1H, m, (CH₃)₂CHCH₂CH), and 0.79 (6H, 2d, J =6.4 Hz, CH(CH₃)₂).; ¹³C NMR: δ (Methanol-d₄) 177.0. 174.1, 173.9, 170.6, 158.0, 132.0, 131.4, 115.7, 68.0, 57.1, 42.6, 42.4, 37.8, 36.8, 26.3, 25.7, 23.5 and 22.3.

### Example 11

[4-(N-Hydroxyamino)-2R-isobutyl-3S-(2-thienylthiomethyl)-succinyl]-L-4-(oxymethylcarboxymethyl)-phenylalanine-N-methylamide (BB 943)

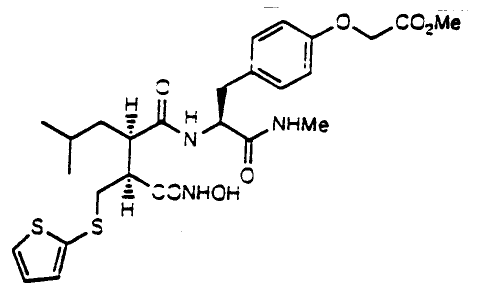

### a) N-Boc-Tyrosine-N-methylamide.

N-Boc-O-benzyltyrosine-N-methylamide (44.3g, 115 mmol) was dissolved in 10% cyclohexene/ethanol (500 mL), 10% palladium on charcoal (4.5g) added and the mixture refluxed for 3 hours. Filtration to remove the catalyst and solvent removal gave the title compound (30.8g, 105 mmol, 91%).

### b) N-Boc-4-(oxymethylcarboxybenzyl)phenylalanine-N-methylamide.

N-Boc-Tyrosine-N-methylamide (30.7g, 104 mmol) was dissolved in acetone (anhydrous, 500 mL), sodium carbonate (13.3g, 125 mmol) and benzylbromoacetate (35.9g, 157 mmol) were added and the mixture refluxed under argon for 4 days. The mixture was filtered, the solvent removed and the residual oil purified by column chromatography using methanol/DCM (2%) as eluant to give the title compound (36.1g, 81.7 mmol, 78%).

### c) N-Boc-4-(oxymethylcarboxymethyl)phenylalanine-N-methylamide.

N-Boc-4-(oxymethylcarboxybenzyl)phenylalanine-N-methylamide (19.7g, 55.8 mmol) was taken up in 10% cyclohexene/ethanol (250 mL), 10% palladium on charcoal (2.6 g) added and the mixture refluxed for one hour. Removal of the catalyst by filtration then evaporation of the solvents gave the acid as a white foam (19.7g, 55.8 mmol 98%). This crude acid was dissolved in DMF (200 mL) and stirred at 0° while potassium carbonate (8.48g, 61 mmol) and methyl iodide (5.21 mL, 83.6 mmol) were added. The mixture was stirred at 0° for 2 hours and at room temperature for one hour. The reaction was filtered to remove inorganic solids, the DMF evaporated under vacuum, then the residue taken up in DCM. The organic layer was washed with brine, dried over magnesium sulphate and finally the solvent removed to give the title ester as a white solid (18.5g, 50.5 mmol, 91%): ¹H-NMR; δ (CDCl₃), 7.11 (2H, d, J= 8.6 Hz, Aryl-H), 6.83 (2H, d, J= 8.6 Hz, Aryl-H), 6.03 (1H, m, NHMe), 5.14 (1H, bd, NHBoc), 4.60 (2H, s, CH₂CO₂Me), 4.25 (1H, q, J=7.0 Hz), 3.80 (3H, s, CO₂CH₃), 2.98 (2H, bd, J=7 Hz, CH₂Ar), 2.71 (3H, d, NHCH₃), and 1.40 (9H, s, C(CH₃)₃).

### d) [4-Benzyloxy-3-benzyloxycarbonyl-2R-isobutylsuccinyl]-L-4-(oxymethylcarboxymethyl)phenylalanine-N-methylamide.

N-Boc-4-(oxymethylcarboxymethyl)phenylalanine-N-methylamide (18.5g, 50.5 mmol) was taken up in DCM/TFA (1:1. 100 mL) and left overnight at 4°. Solvent removal gave the amine as the TFA salt contaminated with excess TFA. This mixture was dissolved in DMF (100 mL), cooled to 0°. NMM (14.6g, 145 mmol) and benzyl (2-benzyloxycarbonyl-5-methyl-3R-pentafluorophenoxycarbonyl)-hexanoate (85.5g, 150 mmol) were added and the reaction stirred at room temperature for 72 hours. The DMF was removed under vacuum and the residual oil taken up in DCM, washed with 2M sodium carbonate, 2M hydrochloric acid and saturated brine then dried over magnesium sulphate. The solution was filtered, the solvent evaporated and the resultant oil purified by column chromatography (silica gel, 30 - 53% ethyl acetate in DCM) to give the title compound as a white foam (17.6 g, 27.3 mmol, 54%): ¹H-NMR; δ (CDCl₃), 7.40-7.17 (10H, m, CO₂CH₂Ph), 7.12 (2H, d, J= 8.6 Hz, Aryl-H), 6.82 (2H, d, J= 8.6 Hz, Aryl-H), 6.67 (1H, d, J=7.8 Hz, NHCH), 5.94 (1H, m NHMe), 5.22 - 5.04 (3H, m, CO₂CH₂Ph and CH(CO₂CH₂Ph)₂), 4.60 (2H, s, CH₂CO₂Me), 4.48 (1H, q, J=7.0 Hz), 3.80 (3H, s, CO₂CH₃), 3.02 - 2.90 (3H, m, CH₂Ar, and (BuCH), 2.65 (3H, d, J=4.7 Hz, NHCH₃), 1.54 (1H, m, (CH₃)₂CHCH₂), 1.35 (1H, m, (CH₃)₂CHCH₂), 1.04 (1H, m, (CH₃)₂CHCH₂), 0.76 (3H, d, J=6.5 Hz, CH(CH₃)₂), and 0.74 (3H, d, J=6.5 Hz, CH(CH₃)₂).

### e) [Hydroxy-2R-isobutyl-3-ethenylsuccinyl]-L-4- (oxymethylcarboxymethyl)-phenylalanine-N-methylamide.

[4-Benzyloxy-3-benzyloxycarbonyl-2R-isobutylsuccinyl]-L-4-(oxymethylcarboxymethyl)phenylalanine-N-methylamide (17.6g 27.3 mmol) was taken up in ethanol (500mL), 10% palladium on charcoal added (3.5g) and the mixture stirred under hydrogen for 2 1/2 hours. The catalyst was removed by filtration and the solvent evaporated to leave the crude malonic acid which was used without further purification. This crude diacid was taken up in ethanol (500 mL), piperidine (2.36g, 27.8 mmol) and formaldehyde (37% aqueous solution, 22.7 mL, 278 mmol) were added and the reaction stirred at room temperature for three days. Solvent removal gave a clear oil which was taken up in ethyl acetate and washed twice with 1M hydrochloric acid and then with brine. The organic layer was separated and dried over magnesium sulphate then the solvent removed to yield the title compound (6.70g, 15.4 mmol, 56%: (¹H-NMR; δ (Methanol-d₄), 7.78 (1H, bd, J=7.2, Hz, CONHCH), 7.10 - 6.75 (5H, m, Aryl-H and NHMe), 6.19 (1H, d, J=3.0 Hz, CH₂=C), 5.58 (1H, d, J=3.6 Hz, CH₂=C), 4.63 (2H, s, CH₂CO₂Me), 4.48 (1H, m, NHCHCO), 3.74 (3H, s, CO₂CH₃), 3.52 (1H, m, ⁱBuCH), 3.00 - 2.78 (2H, m, CH₂Aryl), 2.65 (3H, 2, NHCH₃), 1.63 (1H, m, (CH₃)₂CHCH₂), 1.40 (1H, m, (CH₃)₂CHCH₂), and 0.87 - 0.80 (7H, m + 2xd, J=6.5 Hz, (CH₃)₂CHCH₂ and CH(CH₃)₂).

### f) [4-Hydroxy-2R-isobutyl-3S-(2-thienylthiomethyl)-succinyl]-L-4-(oxymethylcarboxymethyl)-phenylalanine-N-methylamide.

[Hydroxy-2R-isobutyl-3-ethenylsuccinyl]-L-4-(oxymethylcarboxymethyl)-phenylalanine-N-methylamide (4.77g, 11.0 mmol) was taken up in methanol (20 mL) and thiophene-2-thiol (4g) added then the mixture stirred at reflux under argon overnight. Solvent removal gave a yellow solid which was washed twice with diethyl ether to give the title compound as a white solid (1.90g, 3.46 mmol, 31%): ¹H-NMR; δ (DMSO-d₆), 8.35 (1H, bq, NHMe) 7.96 (1H, d, J=8.5, CONHCH), 7.54 (1H, m, Thienyl-H5), 7.18 (2H, d, J=8.5 Hz, Aryl-H), 7.00 (2H, m, Thienyl-H3,4), 6.81 (2H, d, J=8.5 Hz, Aryl-H), 4.71 (2H, s, OCH₂CO₂Me), 4.40 (1H, m, NCHCO), 3.68 (3H, s, CO₂CH₃), 2.95 - 2.64 (2H, m, CH₂Ar), 2.58 (3H, d, J=5.0 Hz, NHCH₃) 2.37 (2H, m, SCH₂CH), 2.14 (1H, s, ¹BuCH), 1.42 (1H, m, (CH₃)₂CHCH₂), 1.23 (1H, m, (CH₃)₂CHCH₂), 0.99 (1H, m, (CH₃)₂CHCH₂), 0.79 (3H, d, J=6 Hz, CH(CH₃)₂), and 0.76 (3H, d, J=6 Hz, CH(CH₃)₂).

### g) [4-(N-Hydroxyamino)-2R-isobutyl-3S-(2-thienylthiomethyl)-succinyl]-L-(4-N-(oxymethylcarboxymethyl)-phenylalanine-N-methylamide.

[4-Hydroxy-2R-isobutyl-3S-(2-thienylthiomethyl)-succinyl]-L-4(oxymethylcarboxymethyl)-phenylalanine-N-methylamide (1.90g, 3.46 mmol) was taken up in DMF (20 mL) and cooled to 0° while HOBT (0.28g, 2.09 mmol), NMM (0.21g, 2.10 mmol) and WSCDI (0.40g, 2.09 mmol) were added. The reaction was stirred at 0° for 3 hours then hydroxylamine hydrochloride (0.22g, 3.22 mmol) and NMM (0.33g, 3.22 mmol), were added and the reaction allowed to warm to room temperature overnight. The DMF was removed under reduced pressure and the resultant crude oil was taken up in diethyl ether/water (1:1) and the resulting solid collected by filtration. This crude solid was purified by recrystallisation from methanol to give the title compound (0.92g, 1.62 mmol, 47%): ¹H-NMR; δ (DMSO-d₆), 10.56 (1H, s, NHOH), 8.89 (1H, s, CONHOH), 8.24 (1H, d, CHCONHCH), 7.79 (1H, m, CONHCH₃), 7.52 (1H, m, Thienyl-H5), 7.17 (2H, d, J=8.4 Hz, Aryl-H), 6.93 (2H, m, Thienyl-H3,4), 6.78 (2H, d, J=8.4 Hz, Aryl-H), 4.69 (2H, s, OCH₂CO₂Me), 4.42 (1H, m, NHCHCO), 3.68 (3H, s, CO₂CH₃), 2.78 (2H, m, CHCH₂Ar), 2.55 (3H, d, J=4.5 Hz, CONHCH₃), 2.48 (2H, m, CHCH₂S and ⁱBuCH), 2.08 (2H, m, CHCH₂S), 1.32 (2H, m, (CH₃)₂CHCH₂), 0.88 (1H, m, (CH₃)₂CHCH₂), 0.78 (3H, d, J=6 Hz, CH(CH₃)₂), and 0.71 (3H, d, J=6 Hz, CH(CH₃)₂): ¹³C NMR: δ (DMSO-d6), 171.9, 170.9, 168.8,
167.7, 155.7, 133.1, 131.8, 130.5, 130.2, 128.7, 127.2, 113.6, 64.2, 53.7, 51.3, 45.6, 45.3, 40.05-38.1, 37.7, 36.1, 25.0, 24.6, 23.6 and 21.06.

### Example 12

[4-(N-Hydroxyamino)-2R-isobutyl-3S-(2-thienylthiomethyl)-succinyl]-L-(4-N(oxymethylcarboxylic acid)-phenylalanine-N-methylamide (BB 944). 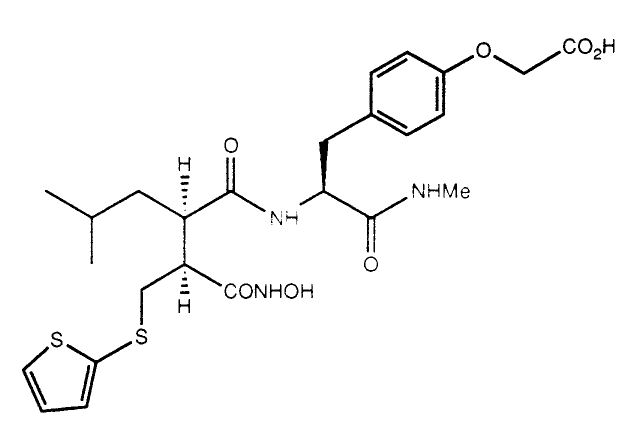
[4-(N-Hydroxyamino)-2R-isobutyl-3S-(2-thienylthiomethyl)succinyl]-L-(4-N-(oxymethylcarboxymethyl)-phenylalanine-N-methylamide (0.42g, 0.75 mmol) was suspended in methanol/water and lithium hydroxide (21 mg, 0.50 mmol) added. After six hours at room temperature the reaction was complete and the solution was neutralised with 1M hydrochloric acid. Removal of the solvents gave the title compound (0.42g, 0.75 mmol, 100%) as a white solid: ¹H-NMR; δ (D₂0), 7.41 (1H, m, Thienyl-H5), 7.22 (2H, d, J=8.4 Hz, Aryl-H), 6.97 (2H, m, Thienyl-H3,4), 6.89 (2H, d, J=8.4 Hz, Aryl-H), 4.48 (1H, m, NHCHCO), 4.40 (2H, s, OCH₂CO₂Me), 3.05 (1H, m, CHCH₂Ar), 2.74 (1H, m, CHCH₂Ar), 2.65 (3H, d, J=4.5 Hz, CONHCH₃), 2.38 (2H, m, CHCH₂S and ⁱBuCH), 2.03 (1H, m, CHCH₂S), 1.78 (1H, m, CHCH₂S), 1.23 (2H, m, (CH₃)₂CHCH₂), 0.98 (1H, m, (CH₃)₂CHCH₂), 0.78 (3H, d, J=6 Hz, CH(CH₃)₂), and 0.71 (3H, d, J=6 Hz, CH(CH₃)₂); ¹³C NMR: δ (D₂O), 176.9, 175.5, 173.6, 168.3, 157.0, 133.3, 123.4, 130.6, 129.9, 128.0, 115.0, 66.9, 55.4, 46.7, 46.0, 40.0, 36.6, 36.4, 26.0, 25.6, 23.2 and 20.8.

### Example 13

[4-(N-Hydroxyamino)-2R-isobutyl-3S-(2-thienylthiomethyl)succinyl]-L-4-(oxymethylcarboxyglycyl methyl ester)-phenylalanine-N-methylamide (B888)

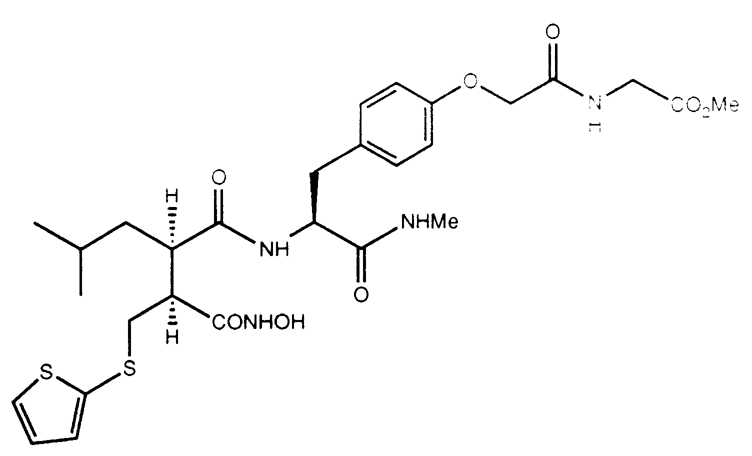

### a) N-Boc-4-(oxymethylcarboxylic acid)-phenylalanine-N-methylamide

N-Boc-4-(oxymethylcarboxybenzyl)phenylalanine-N-methylamide (35.5g, 80.3 mmol) was dissolved in 20% cyclohexene in ethanol (400 mL), 7.1g 10% palladium on charcoal added and the mixture heated under reflux for 15 minutes. The catalyst was removed by filtration and the solvent evaporated to leave the title compound (28.5g, 80.5 mmol, 100%): ¹H-NMR; δ (Methanol-d₄), 7.09 (2H, d, J=8.6 Hz, Aryl-H), 6.81 (2H, d, J=8.6 Hz, Aryl-H), 4.56 (2H, s, OCH₂CO₂H), 4.18 (1H, m, NHCHCO), 2.95 - 2.72 (2H, m, CH₂Ar), 2.62 (3H, s, CONHCH₃), and 1.32 (9H, s, C(CH₃)₃).

### b) N-Boc-4-(oxymethylcarboxyglycyl methyl ester)-phenylalanine-N-methylamide.

N-Boc-4-(oxymethylcarboxylic acid)-phenylalanine-N-methylamide (7.47g, 21.2 mmol) was taken up in DMF (25 mL) and cooled to 0°. To this solution was added, pentafluorophenol (7.80g, 42.4 mmol), glycine methyl ester (from the hydrochloride salt, 3.19g, 25.4 mmol) and WSCDI (4.88g, 25.4 mmol) and the mixture stirred at room temperature overnight. The solvent was removed by evaporation and the residual oil taken up in DCM which was then sequentially washed with 2M sodium carbonate solution, 2M hydrochloric acid solution and saturated brine. The separated organic layer was dried over magnesium sulphate and the solvent evaporated to leave the title compound (7.08g, 16.7 mmol, 79%): ¹H-NMR; δ (CDCl₃), 7.12 (2H, d, J=8.6 Hz, Aryl-H), 6.84 (2H, d, J=8.6 Hz, Aryl-H), 6.11 (1H, bq, CONHMe), 5.17 (1H, bd, CONHCH), 4.48 (2H, s, OCH₂CO₂H), 4.28 (1H, m, NHCHCO), 4.12 (2H, bd, NHCH₂CO₂Me), 3.76 (3H, s, CO₂CH₃), 2.96 (2H, m, CH₂Ar), 2.72 (3H, d, J=4.8 Hz, CONHCH₃), and 1.38 (9H, s, C(CH₃)₃).

### c) [4-Benzyloxy-3-benzyloxycarbonyl-2R-isobutylsuccinyl]-L-4-(oxymethylcarboxyglycyl methyl ester)phenylalanine-N-methylamide.

N-Boc-4-(oxymethylcarboxyglycyl methyl ester)-phenylalanine-N-methylamide (5.35g, 12.6 mmol) was dissolved in TFA/DCM (1:1, 100 mL) and stood overnight at 4°. Solvent removal gave an oil which was dissolved in DCM and washed with 2M sodium carbonate, dried over magnesium sulphate and the solvent removed to leave the free amine as a yellow solid (3.71g, 11.1 mmol, 88%). This was coupled with benzyl (2-benzyloxycarbonyl-5-methyl-3R-pentafluorophenoxycarbonyl)-hexanoate (12.4g, 21.9 mmol) as described in example 11d to give the title compound (6.60g, 9.38 mmol, 86%): ¹H-NMR: δ (CDCl₃), 7.35 - 7.20 (10H, m, Ph), 7.15 (2H, d, J=8.6 Hz, Aryl-H), 6.84 (2H, d, J=8.6 Hz, Aryl-H), 6.67 (1H, bd, CONHCH), 5.92 (1H, bq, CONHMe), 5.17 - 5.05 (4H, m, CH(CO₂CH₂Ph)₂), 4.48 (3H, s + m, OCH₂CO₂H and NHCHCO), 4 12 (2H, bd, NHCH₂CO₂Me), 3.80 (1H, d, J=9.3 Hz, CH(CO₂CH₂Ph)₂), 3.76 (3H, s, CO₂CH₃), 2.97 (2H, m, CH₂Ar), 2.67 (3H, d, J=4.8 Hz, CONHCH₃), 1.54 (1H, m, (CH₃)₂CHCH₂), 1.35 (1H, m, (CH₃)₂CHCH₂), 1.02 (1H, m, (CH₃)₂CHCH₂), 0.76 (3H, d, J=6.4 Hz, CH(CH₃)₂), and 0.75 (3H, d, J=6.4 Hz, CH(CH₃)₂).

### d) [Hydroxy-2R-isobutyl-3-ethenylsuccinyl]-L-4-(oxymethylcarboxyglycyl methyl ester)-phenylalanine-N-methylamide

[4-Benzyloxy-3-benzyloxycarbonyl-2R-isobutylsuccinyl]-L-4-(oxymethylcarboxyglycyl methyl ester)phenylalanine-N-methylamide (7.62g, 10.8 mmol) was treated as described in example 11e to yield the title compound (3.77g, 7.67 mmol, 72%): ¹H-NMR; δ (Methanol-d₄), 7.80 (2H, bm, NH), 7.08 (2H, d, J=8.7 Hz, Aryl-H), 6.86 (2H, d, J=8.7 Hz, Aryl-H), 6.18 (1H, d, J= 3.0 Hz, CH₂=C), 5.59 (1H, d, J = 3.6 Hz, CH₂=C), 4.49 (2H, s, OCH₂CO₂H), 4.45 (1H, m, NHCHCO), 3.99 (2H, s, NHCH₂CO₂Me), 3.69 (3H, s, CO₂CH₃), 3.52 (1H, bm, CHCO₂H), 2.88 (2H, m, CH₂Ar), 2.65 (3H, d, J=4.8 Hz, CONHCH₃), 1.63 (1H, m, (CH₃)₂CHCH₂), 1.40 (2H, m, (CH₃)₂CHCH₂), 0.86 (3H, d, J=6.2 Hz, CH(CH₃)₂), and 0.81 (3H, d, J=6.3 Hz, CH(CH₃)₂).

### e) [4-Hydroxy-2R-isobutyl-3S-(2-thienylthiomethyl)-succinyl]-L-4-(oxymethylcarboxyglycyl methyl ester)-phenylalanine-N-methylamide.

[Hydroxy-2R-isobutyl-3-ethenylsuccinyl]-L-4-(oxymethylcarboxyglycyl methyl ester)-phenylalanine-N-methylamide (1.56g, 3.17 mmol) was treated with thiophene-2-thiol as described in example 11f to give the title compound as a white solid (1.30g, 2.14 mmol. 67%): ¹H-NMR; δ (Methanol-d₄), 8.39 (1H, bd, J=8.5 Hz, NHCH), 7.85 (1H, m, CONHMe), 7.38 (1H, dd, J=5.3, 1.3 Hz, Thienyl-H5), 7.21 (2H, d, J=8.6 Hz, Aryl-H), 6.98 (1H, dd, J=3.5, 1.3 Hz, Thienyl-H3), 6.94 (2H, d, J=8.7 Hz, Aryl-H), 6.91 (1H, dd, J=5.2, 3.5 Hz, Thienyl-H4), 4.55 (3H, s, OCH₂CO₂H and NHCHCO), 3.96 (2H, s, NHCH₂CO₂Me), 3.67 (3H, s, CO₂CH₃), 2.91 (1H, dd, J= 13.9, 5.1 Hz, CH₂Ar), 2.76 (1H, dd, J= 13.9, 5.1 Hz, CH₂Ar), 2.66 (3H, s, CONHCH₃), 2.45 (3H, m, SCH₂CH and CHCO₂H), 2.16 (1H, m, SCH₂CH), 1.50 (1H, m, (CH₃)₂CHCH₂), 1.30 (1H, m, (CH₃)₂CHCH₂), 0.98 (1H, m, (CH₃)₂CHCH₂), 0.83 (3H, d, J=6.5 Hz, CH(CH₃)₂), and 0.76 (3H, d, J=6.3 Hz, CH(CH₃)₂).

### f) [4-(N-Hydroxyamino)-2R-isobutyl-3S-(2-thienylthiomethyl)-succinyl]-L-4-(oxymethylcarboxyglycyl methyl ester)-phenylalanine-N-methylamide

[4-Hydroxy-2R-isobutyl-3S-(2-thienylthiomethyl)-succinyl]-L-4-(oxymethylcarboxyglycyl methyl ester)-phenylalanine-N-methylamide (1.29g, 2.12 mmol) was treated as described in example 11g to give the title compound as a white solid (0.90g, 1.45 mmol, 68%):Anal. calculated for C₂₈H₃₈N₄O₈S₂, Requires, C 54.00, H 6.15, N 9.00; Found, C 54.04, H 6.16, N 8.79: ¹H-NMR; δ (DMSO-d₆), 10.55 (1H, s, CONHOH), 8.89 (1H, s, CONHOH), 8.45 (1H, m, CONHCH₂), 8.23 (1H, bd, J=8.5 Hz, NHCH), 7.79 (1H, m, CONHMe), 7.51 (1H, dd, J=5.3, 1.3 Hz, Thienyl-H5), 7.19 (2H, d, J=8.6 Hz, Aryl-H), 6.95 (2H, m, Thienyl-H3,4), 6.83 (2H, d, J=8.5 Hz, Aryl-H), 4.47 (2H, s, OCH₂CO₂Me), 4.45 (1H, m, NHCHCO), 3.92 (2H, s, NHCH₂CO₂Me), 3.63 (3H, s, CO₂CH₃), 2.88 (1H, m, SCH₂CH), 2.62 (2H, m, CH₂Ar), 2.53 (3H, d, J=4.5 Hz, CONHCH₃), 2.39 (1H, m, ⁱBuCH), 2.16 (1H, m, SCH₂CH), 1.99 (1H, m, SCH₂CH), 1.31 (2H, m, (CH₃)₂CHCH₂), 0.83 (1H, m, (CH₃)₂CHCH₂), 0.80 (3H, d, J=6.6 Hz, CH(CH₃)₂), and 0.76 (3H, d, J=6.6 Hz, CH(CH₃)₂) ¹³C-NMR: δ (DMSO-d₆, 172.4, 171.4, 170.0, 168.3, 167.9, 156.1, 132.3, 130.7, 130.1, 129.2, 127.7, 114.4, 66.8, 54.2, 51.7, 46.0, 45.8, 40.5-38.5, 38.1, 36.6, 25.4, 25.0, 24.0 and 21.5

### Example 14

[4-(N-Hydroxyamino)-2R-isobutyl-3S-(2-thienylthiomethyl)-succinyl]-L-4-(oxymethylcarboxyglycine)-phenylalanine-N-methylamide (BB 899). 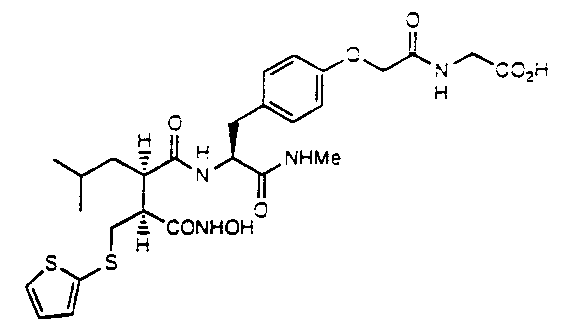
[4-(N-Hydroxyamino)-2R-isobutyl-3S-(2-thienylthiomethyl)-succinyl]-L-4-(oxymethylcarboxyglycyl methyl ester)-phenylalanine-N-methylamide (0.45g, 0.73 mmol) was treated with lithium hydroxide as described in example to give the title compound (0.41g, 0.67 mmol, 92%): ¹H-NMR: δ (D₂O), 7.43 (1H, dd, J=5.3, 1.3 Hz, Thienyl-H5), 7.28 (2H, d, J=8.5 Hz, Aryl-H), 7.06 (2H, d, J=8.5 Hz, Aryl-H), 6.95 (1H, m, Thienyl-H3), 6.83 (1H, m, Thienyl-H4), 4.70 (1H, m, NHCHCO), 4.58 (2H, s, NHCH₂CONH), 3.63 (2H, bs, NHCH₂CO₂H), 3.12 (1H, m, CH₂Ar), 2.79 (1H, m, CH₂Ar), 2.68 (3H, s, CONHCH₃), 2.21 (2H, m, SCH₂CH and ⁱBuCH), 2.05 (1H, m, SCH₂CH), 1.65 (1H, m, SCH₂CH), 1.19 (2H, m, (CH₃)₂CHCH₂), 0.91 (1H, m, (CH₃)₂CHCH₂), 0.81 (3H, d, J=6.6 Hz, CH(CH₃)₂), and 0.76 (3H, d, J=6.6 Hz, CH(CH₃)₂): ¹³C-NMR; δ (D₂O), 195.6, 176.6, 175.5, 173.8, 171.6, 156.7, 132.8, 132.2, 130.8, 130.6, 129.7, 128.1, 115.5, 66.9, 55.3, 46.8, 46.0, 42.8, 40.0, 36.7, 36.3, 26.5, 26.1, 23.5 and 20.1.

### Example 15

[4-(N-Hydroxyamino)-2R-isobutyl-3S-methyl-succinyl]-L-4-(oxymethylcarboxyglycyl methyl ester)-phenylalanine-N-methylamide (BB 877)

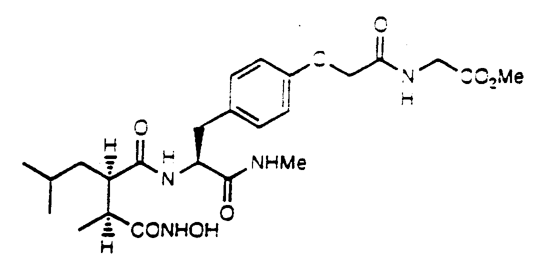

### a) [4-(N-Benzyloxyamino)-2R-isobutyl-3S-methylsuccinyl]-L-4-(oxymethylcarboxyglycyl methyl ester)-phenylalanine-N-methylamide

[Hydroxy-2R-isobutyl-3-ethenylsuccinyl]-L-4-(oxymethylcarboxyglycyl methyl ester)-phenylalanine-N-methylamide (1.05g, 2.14 mmol) was dissolved in ethanol (40 mL), 10% palladium on charcoal added and the mixture subjected to an atmosphere of hydrogen for one hour. The catalyst was removed by filtration and solvent removal gave the saturated compound as a solid (0.99g, 2.00 mmol. 93%). This material (0.97g, 1.96 mmol) was dissolved in DMF/DCM (20%, 10 mL), pentafluorophenol (0.72g, 3.93 mmol), O-benzylhydroxylamine (0.48g, 3.93 mmol), NMM (0.26g, 2.55 mmol) and WSCDI (0.49g, 2.55 mmol) added and the reaction mixture stirred overnight. The preciptated solid was filtered off and the solvent removed from the filtrate to leave a solid which was washed with 1M hydrochloric acid and ethyl acetate. The combined solids were recrystallised from methanol to give the title benzhydroxamate (0.42g, 0.70 mmol, 36%): ¹H-NMR: δ (DMSO-d₆), 10.98 (1H, s, CONHOBn), 8.48 (1H, m, NHCH₂), 8.15 (1H, d, J=9 Hz, NHCH) 7.73 (1H, m, CONHMe) 7.36 (5H, s, Ph), 7.19 (2H, d, J=8.6 Hz, Aryl-H), 6.86 (2H, d, J=8.7 Hz, Aryl-H), 4.74 (2H, s, CH₂Ph), 4.46 (3H, s, OCH₂CO₂Me and NHCHCO), 3.90 (2H, d, J=5.6 Hz, NHCH₂CO₂Me), 3.63 (3H, s, CO₂CH₃), 2.89 (1H, m, CH₂Ar), 2.73 (1H, m, CH₂Ar), 2.56 (3H, d, J=4.5 Hz, CONHCH₃), 2.37 (1H, m, CHCONHOBn), 1.91 (1H, m, ⁱBuCH), 1.30 (2H, m, (CH₃)₂CHCH₂), 0.83 (3H, d, J=6.3 Hz, CH(CH₃)₂), 0.72 (3H, d, J=6.3 Hz, CH(CH₃)₂), 0.71 (1H, m, (CH₃)₂CHCH₂), and 0.45 (3H, d, J=6.6 Hz, CHCH₃).

### b) [4-(N-Hydroxyamino)-2R-isobutyl-3S-methyl-succinyl]-L-4-(oxymethylcarboxyglycyl methyl ester)-phenylalanine-N-methylamide

[4-(N-Benzyloxyamino)-2R-isobutyl-3S-methyl-succinyl]-L-4-(oxymethylcarboxyglycyl methyl ester)-phenylalanine-N-methylamide (0.42g, 0.70 mmol) was hydrogenated as described in example 10c to give the title compound as a solid (0.29g, 0.57 mmol, 82%): Anal. calculated for C₂₄H₃₆N₄O₈: Requires. C 56.86, H 7.13, N 11.02: Found. C 56.14, H 7.08, N 10.60: ¹H-NMR; δ (DMSO-d₆), 8.70 (1H, s, CONHOH), 8.49 (1H, m, NHCH₂), 8.18 (1H, d, J=8.5 Hz, NHCH), 7.73 (1H, m, CONHMe), 7.19 (2H, d, J=8.5 Hz, Aryl-H), 6.85 (2H, d, J=8.6 Hz, Aryl-H), 4.47 (1H, m, NHCHCO), 4.48 (2H, s, OCH₂CO₂Me), 3.89 (2H, d, J=5.9 Hz, NHCH₂CO₂Me), 3.63 (3H, s, CO₂CH₃), 2.89 (1H, m, CH₂Ar), 2.69 (1H, m, CH₂Ar), 2.58 (3H, d, J=4.5 Hz, CONHCH₃), 2.39 (1H, m, CHCONHOBn), 1.98 (1H, m, ⁱBuCH) 1.32 (2H, m, (CH₃)₂CHCH₂), 0.88 (1H, m, (CH₃)₂CHCH₂), 0.83 (3H, d, J=6.5 Hz, CH(CH₃)₂), 0.72 (3H, d, J=6.5 Hz, CH(CH₃)₂), and 0.48 (3H, d, J=6.6 Hz, CHCH₃): ¹³C-NMR: δ (DMSO-d₆), 172.8, 171.1, 170.7, 169.6, 167.9, 155.7, 130.6, 129.7, 113.9, 66.6, 53.7, 51.3, 46.2, 39.9-38.0, 36.1, 25.0, 24.8, 23.6, 21.1 and 15.6.

### Example 16

[4-(N-Hydroxyamino)-2R-isobutyl-3S-methylsuccinyl]-L-4-(oxymethylcarboxyglyine)-phenylalanine-N-methylamide 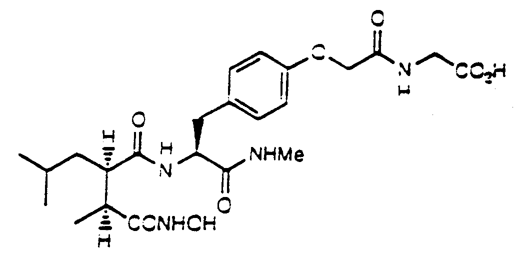
[4-(N-Hydroxyamino)-2R-isobutyl-3S-methyl-succinyl]-L-4-(oxymethylcarboxyglycyl methyl ester)-phenylalanine-N-methylamide (0.11g, 0.22 mmol) was treated with lithium hydroxide as described in example 12 to give the title compound (0.09g, 0.18 mmol, 83%): ¹H-NMR; δ (D₂O), 7.19 (2H, d, J=8.5 Hz, Aryl-H), 6.92 (2H, d, J=8.6 Hz, Aryl-H), 4.56 (2H, s, OCH₂CO₂Me), 4.51 (1H, m, NHCHCO), 3.80 (2H, s, NHCH₂CO₂Me), 2.96 (2H, m, CH₂Ar), 2.61 (3H, s, CONHCH₃), 2.42 (1H, m, CHCONHOBn), 2.11 (1H, m, ⁱBuCH), 1.31 (2H, m, (CH₃)₂CHCH₂), 0.95 (1H, m, (CH₃)₂CHCH₂), 0.83 (3H, d, J=6.5 Hz, CH(CH₃)₂), 0.75 (3H, d, J=6.5 Hz, CH(CH₃)₂), and 0.61 (3H, d, J=6.6 Hz, CHCH₃): ¹³C-NMR: δ (D₂O), 195.6, 176.6, 175.5, 173.8, 171.6, 156.7, 132.8, 132.2, 130.8, 130.6, 129.7, 128.1, 115.5, 66.9, 55.3, 46.8, 46.0, 42.8, 40.0, 36.7, 36.3, 26.5, 26.1, 23.5 and 20.1.

## Claims

1. A compound of general formula (I): 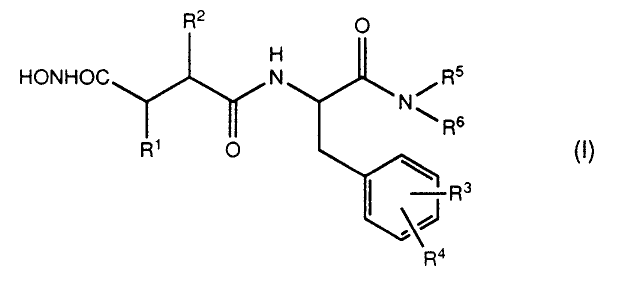 wherein
R¹ is hydrogen; C₁-C₆ alkyl; phenyl; phenyl substituted by up to four subsituents each of which may independently be C₁-C₆ alkyl, C₁-C₆ alkoxy, hydroxy, thiol, C₁-C₆ alkylthiol, amino, halo, trifluoromethyl, nitro, -COOH, -COONH₂, or -CONHR^{A} wherein R^{A} represents a C₁-C₆ alkyl group or the residue of an amino acid selected from alanine, valine, leucine, isoleucine, phenylalanine, tryptophan, methionine, glycine, serine, threonine, cysteine, tyrosine, asparagine, glutamine, aspartic acid, glutaminic acid and histidine; phenyl (C₁-C₆ alkyl) or heterocyclyl;
or R¹ is -ASOₙR⁷, wherein A represents a C₁-C₆ hydrocarbon chain optionally substituted with one or more C₁-C₆ alkyl groups, phenyl groups, or phenyl groups substituted by up to four substituents as specified above; n = 0, 1 or 2; and R⁷ is C₁-C₆ alkyl; phenyl; phenyl substituted by up to four substituents as specified above; phenyl (C₁-C₆ alkyl, heterocyclyl, (C₁-C₆ alkyl)acyl, thienyl or phenacyl;
R² is hydrogen, C₁-C₆ alkyl, C₂-C₆ alkenyl, phenyl(C₁-C₆ alkyl), or C₃-C₈ cycloalkyl(C₁-C₆ alkyl);
R⁵ is hydrogen or C₁-C₆ alkyl, or (C₁-C₆ alkyl)phenyl;
R⁶ is hydrogen or methyl;
characterised in that
R⁴ is hydrogen and
R³ is -O-CH₂-CO-R⁸ where R⁸ is hydroxyl; (C₁-C₆)alkoxy; phenyl (C₁-C₆)alkoxy; amino; (C₁-C₆)alkylamino; di((C₁-C₆)alkyl)amino; phenyl (C₁-C₆)alkylamino; the residue of an amino acid or acid halide, ester or amide derivative thereof, said residue being linked via an amide bond, said amino acid being selected from glycine, α or β alanine, valine, leucine, isoleucine, phenylalanine, tyrosine, tryptophan, serine, threonine, cysteine, methionine, asparagine, glutamine, lysine, histidine, arginine, glutamic acid, and aspartic acid;
or a salt thereof.

2. A compound as claimed in claim 1, in which the chiral centre adjacent to the substituent R² has R stereochemistry.

3. A compound as claimed in claim 1 or claim 2, in which the chiral centre adjacent to the substituted benzyl group has S stereochemistry.

4. A compound as claimed in any one of claims 1 to 3, in which the chiral centre adjacent to the substituent R¹ has S stereochemistry.

5. A compound as claimed in any one of claims 1 to 4 in which R¹ represents a hydrogen atom or a C₁-C₄ alkyl group, or an arythiomethyl group or a thiophenethiomethyl group.

6. A compound as claimed in any one of claims 1 to 5 in which R² represents a C₃-C₆ alkyl group.

7. A compound as claimed in any one of claims 1 to 6 in which R⁵ represents a C₁-C₅ alkyl group.

8. A compound as claimed in any one of claims 1 to 7 in which R⁶ represents a hydrogen atom.

9. [4-(N-Hydroxamino)-2R-isobutylsuccinyl]-L-(4-oxymethylcarboxylic acid)phenylalanine-N-methylamide;
[4-(N-Hydroxamino)-2R-isobutylsuccinyl]-L-(4-oxymethylcoarboxy-N-methylamide)-phenylalanine-N-methylamide;
[4-(N-Hydroxamino)-2R-isobutylsuccinyl]-L-(4-oxymethylcarboxy-beta-alanine)-phenylalanine-N-methylamide;
[4-(N-Hydroxamino)-2R-isobutylsuccinyl]-L-(4-oxymethylcarboxyglycine)phenylalanine-N-methylamide;
[4-(N-Hydroxamino)-2R-isobutylsuccinyl]-L-(4-oxymethylcarboxy-N-benzylamide)phenylalanine-N-methylamide;
[4-(N-Hydroxamino)-2R-isobutyl-3S-(2-thienylthiomethyl)succinyl]-L-(4-oxymethylcarboxymethyl)-phenylalanine-N-methylamide;
[4-(N-Hydroxamino)-2R-isobutyl-3S-(2-thienylthiomethyl)succinyl]-L-(4-oxymethylcarboxylic acid)-phenylalanine-N-methylamide;
[4-(N-Hydroxamino)-2R-isobutyl-3S-(2-thienylthiomethyl)succinyl]-L-(4-oxymethylcarboxyglycyl methyl ester)-phenylalanine-N-methylamide;
[4-(N-Hydroxamino)-2R-isobutyl-3S-(2-thienylthiomethyl)succinyl]-L-(4-oxymethylcarboxyglycine)-phenylalanine-N-methylamide;
[4-(N-Hydroxamino)-2R-isobutyl-3S-methylsuccinyl]-L-(4-oxymethylcarboxyglycyl methyl ester)-phenylalanine-N-methylamide;
[4-(N-Hydroxamino)-2R-isobutyl-3S-methylsuccinyl]-L-(4-oxymethylcarboxyglycine)-phenylalanine-N-methylamide;
or a salt of one of them.

10. A compound as claimed in any one of claims 1 to 9 for use in human or veterinary medicine.

11. The use of a compound as claimed in any one of claims 1 to 9 in the preparation of an agent for use in the management of disease involving collagen breakdown.

12. A pharmaceutical or veterinary composition comprising a compound as claimed in any one of claims 1 to 9 together with a pharmaceutically and/or veterinarily acceptable carrier.

## Patentansprüche

1. Verbindung der allgemeinen Formel (I): 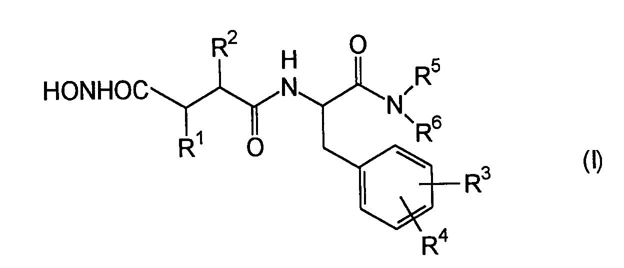 worin R¹ Wasserstoff, C₁₋₆-Alkyl, Phenyl, mit 1 bis 4 Substituenten substituiertes Phenyl, die jeweils unabhängig voneinander C₁₋₆-Alkyl, C₁₋₆-Alkoxy, Hydroxy, Thiol, C₁₋₆-Alkylthiol, Amino, Halogen, Trifluormethyl, Nitro, -COOH, -COONH₂ oder -CONHR^{A}, worin R^{A} eine C₁₋₆-Alkylgruppe oder einen Aminosäurerest, ausgewählt aus Alanin, Valin, Leucin, Isoleucin, Phenylalanin, Tryptophan, Methionin, Glycin, Serin, Threonin, Cystein, Tyrosin, Asparagin, Glutamin, Asparaginsäure, Glutaminsäure und Histidin, repräsentiert, sein können, Phenyl(C₁₋₆-alkyl) oder Heterocyclyl ist; oder
R¹ ist -ASOₙR⁷, worin A eine C₁₋₆-Kohlenwasserstoffkette repräsentiert, die wahlweise mit einer oder mehreren C₁₋₆-Alkylgruppen, Phenylgruppen oder mit bis zu 4 wie oben spezifizierten Substituenten substituierte Phenylgruppen substituiert sein können; n = 0, 1 oder 2 und R⁷ ist C₁₋₆-Alkyl, Phenyl, mit bis zu 4 wie oben spezifizierten Substituenten substituiertes Phenyl, Phenyl(C₁₋₆-alkyl), Heterocyclyl, (C₁₋₆-Alkyl)acyl, Thienyl oder Phenacyl;
R² ist Wasserstoff, C₁₋₆-Alkyl, C₂₋₆-Alkenyl, Phenyl(C₁₋₆-alkyl) oder C₃₋₈-Cycloalkyl(C₁₋₆-alkyl);
R⁵ ist Wasserstoff oder C₁₋₆-Alkyl oder (C₁₋₆-Alkyl)phenyl;
R⁶ ist Wasserstoff oder Methyl;
dadurch **gekennzeichnet,** dass
R⁴ Wasserstoff ist, und
R³ ist -O-CH₂-CO-R⁸, worin R⁸ Hydroxyl, (C₁₋₆)Alkoxy, Phenyl(C₁₋₆-alkoxy), Amino, (C₁₋₆)Alkylamino, Di(C₁₋₆-alkyl)amino, Phenyl(C₁₋₆)alkylamino, ein Aminosäurerest oder ein Säurehalogenid, Ester oder Amidderivat davon, wobei der Rest über eine Amidbindung gebunden ist, die Aminosäure ist ausgewählt aus Glycin, α- oder β-Alanin, Valin, Leucin, Isoleucin, Phenylalanin, Tyrosin, Tryptophan, Serin, Threonin, Cystein, Methionin, Asparagin, Glutamin, Lysin, Histidin, Arginin, Glutaminsäure und Asparaginsäure,
oder ein Salz davon.

2. Verbindung gemäss Anspruch 1, worin das dem Substituenten R² benachbarte chirale Zentrum R-Konfiguration besitzt.

3. Verbindung gemäss Anspruch 1 oder 2, worin das der substituierten Benzylgruppe benachbarte chirale Zentrum S-Konfiguration besitzt.

4. Verbindung gemäss mindestens einem der Ansprüche 1 bis 3, worin das dem Substituenten R¹ benachbarte chirale Zentrum die S-Konfiguration besitzt.

5. Verbindung gemäss mindestens einem der Ansprüche 1 bis 4, worin R¹ ein Wasserstoffatom oder eine C₁₋₄-Alkylgruppe oder eine Arylthiomethylgruppe oder eine Thiophenethiomethylgruppe repräsentiert.

6. Verbindung gemäss mindestens einem der Ansprüche 1 bis 5, worin R² eine C₃₋₆-Alkylgruppe repräsentiert.

7. Verbindung gemäss mindestens einem der Ansprüche 1 bis 6, worin R⁵ eine C₁₋₅-Alkylgruppe repräsentiert.

8. Verbindung gemäss mindestens einem der Ansprüche 1 bis 7, worin R⁶ ein Wasserstoffatom repräsentiert.

9. [4-(N-Hydroxamino)-2R-isobutylsuccinyl]-L-(4-oxymethylcarbonsäure)phenylalanin-N-methylamid,
[4-(N-Hydroxamino)-2R-isobutylsuccinyl]-L-(4-oxymethylcarboxy-N-methylamid)phenylalanin-N-methylamid,
[4-(N-Hydroxamino)-2R-isobutylsuccinyl]-L-(4-oxymethylcarboxy-β-alanin)phenylalanin-N-methylamid,
[4-(N-Hydroxamino)-2R-isobutylsuccinyl]-L-(4-oxymethylcarboxyglycin)phenylalanin-N-methylamid,
[4-(N-Hydroxamino)-2R-isobutylsuccinyl]-L-(4-oxymethylcarboxy-N-benzylamid)phenylalanin-N-methylamid,
[4-(N-Hydroxamino)-2R-isobutyl-3S-(2-thienylthiomethyl)succinyl]-L-(4-oxymethylcarboxymethyl)-phenylalanin-N-methylamid,
[4-(N-Hydroxamino)-2R-isobutyl-3S-(2-thienylthiomethyl)succinyl]-L-(4-oxymethylcarbonsäure)phenylalanin-N-methylamid,
[4-(N-Hydroxamino)-2R-isobutyl-3S-(2-thienylmethyl)succinyl]-L-(4-oxymethylcarboxyglycylmethylester)phenylalanin-N-methylamid,
[4-(N-Hydroxamino)-2R-isobutyl-3S-(2-thienylthiomethyl)succinyl]-4-oxymethylcarboxyglycin)phenylalanin-N-methylamid,
[4-(N-Hydroxamino)-2R-isobutyl-3S-methylsuccinyl]-L-(4-oxymethylcarboxyglycylmethylester)phenylalanin-N-methylamid,
[4-(N-Hydroxamino)-2R-isobutyl-3S-methylsuccinyl]-L-(4-oxymethylcarboxyglycin)phenylalanin-N-methylamid,
oder ein Salz von einer dieser Verbindungen.

10. Verbindung gemäss mindestens einem der Ansprüche 1 bis 9 zur Verwendung in der Human- oder Tiermedizin.

11. Verwendung einer Verbindung gemäss mindestens einem der Ansprüche 1 bis 9 zur Herstellung eines Mittels zur Verwendung in der Behandlung von mit Kollagenstörungen verbundenen Krankheiten.

12. Pharmazeutische oder tiermedizinische Zusammensetzung, umfassend eine Verbindung gemäss mindestens einem der Ansprüche 1 bis 9 zusammen mit einem pharmazeutisch und/oder tiermedizinisch annehmbaren Träger.

## Revendications

1. Composé de formule générale (I) : 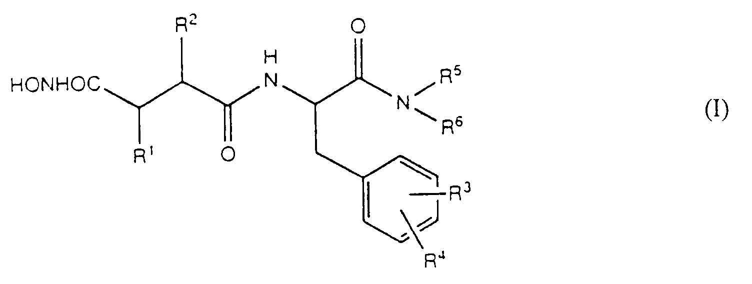 dans laquelle :
R¹ est l'hydrogène, un groupe alkyle en C₁-C₆ ; phényle; phényle substitué par jusqu'à quatre substituants, chacun d'entre eux pouvant être indépendamment un groupe alkyle en C₁-C₆, alcoxy en C₁-C₆, hydroxy, thio, (alkyl en C₁-C₆)thio amino, halogéno, trifluorométhyle, nitro, -COOH, -COONH₂ ou -CONHR^{A} où R^{A} représente un groupe alkyle en C₁-C₆ ou le résidu d'un acide aminé choisi parmi l'alanine, la valine, la leucine, l'isoleucine, la phénylalanine, le tryptophane, la méthionine, la glycine, la sérine, la thréonine, la cystéine, la tyrosine, l'asparagine, la glutamine, l'acide aspartique, l'acide glutamique et l'histidine ; phényl(alkyle en C₁-C₆) ou hétérocyclyle;
ou bien R¹ est un groupe -ASOₙR⁷ où A représente une chaîne hydrocarbonée en C₁-C₆ éventuellement substituée par un ou plusieurs groupes alkyle en C₁-C₆, groupes phényle ou groupes phényle substitués par jusqu'à quatre substituants tels qu'indiqués ci-dessus ; n vaut 0, 1 ou 2 ; et R⁷ est un groupe alkyle en C₁-C₆ ; phényle ; phényle substitué par jusqu'à quatre substituants tels qu'indiqués ci-dessus ; phényl(alkyle en C₁-C₆), hétérocyclyle (alkyl en C₁-C₆)acyle, thiényle ou phénacyle ;
R² est l'hydrogène ou un groupe alkyle en C₁-C₆, alcényle en C₂-C₆, phényl(alkyle en C₁-C₆) ou (cycloalkyl en C₃-C₈)-(alkyle en C₁-C₆) ;
R⁵ est l'hydrogène ou un groupe alkyle en C₁-C₆ ou (alkyl en C₁-C₆)phényle ;
R⁶ est l'hydrogène ou un groupe méthyle ;
caractérisé en ce que :
R⁴ est l'hydrogène, et
R³ est un groupe -O-CH₂-CO-R⁸ où R⁸ est un groupe hydroxyle ; alcoxy en C₁-C₆ ; phényl(alcoxy en C₁-C₆) ; amino ; (alkyl en C₁-C₆)amino ; di(alkyl en C₁-C₆)amino ; phényl(alkyl en C₁-C₆)amino ; le résidu d'un acide aminé ou d'un halogénure d'acide, ou encore d'un dérivé ester ou amide de celui-ci, ledit résidu étant lié par l'intermédiaire d'une liaison amide, ledit acide aminé étant choisi parmi la glycine, l'α-alanine, la β-alanine, la valine, la leucine, l'isoleucine, la phénylalanine la tyrosine le tryptophane, la sérine, la thréonine, la cystéine, la méthionine, l'asparagine, la glutamine, la lysine, l'histidine, l'arginine, l'acide glutamique et l'acide aspartique ;
ou un sel d'un tel composé.

2. Composé selon la revendication 1, dans lequel le centre chiral adjacent au substituant R² a une stéréochimie R.

3. Composé selon la revendication 1 ou 2, dans lequel le centre chiral adjacent au groupe benzyle substitué a une stéréochimie S.

4. Composé selon l'une quelconque des revendications 1 à 3, dans lequel le centre chiral adjacent au substituant R¹ a une stéréochimie S.

5. Composé selon l'une quelconque des revendications 1 à 4, dans lequel R¹ représente un atome d'hydrogène ou un groupe alkyle en C₁-C₄, un groupe arylthiométhyle ou un groupe thiophène-thiométhyle.

6. Composé selon l'une quelconque des revendications 1 à 5, dans lequel R² représente un groupe alkyle en C₃-C₆.

7. Composé selon l'une quelconque des revendications 1 à 6, dans lequel R⁵ représente un groupe alkyle en C₁-C₅.

8. Composé selon l'une quelconque des revendications 1 à 7, dans lequel R⁶ représente un atome d'hydrogène.

9. [4 - (N-hydroxyamino) - 2R - isobutylsuccinyl ] - L - (acide 4-oxyméthyl-carboxylique)phénylalanine-N-méthylamide ;
[4-(N-hydroxyamino)-2R-isobutylsuccinyl]-L-(4-oxyméthylcarboxy-N-méthylamide)phénylalanine-N-méthylamide ;
[4-(N-hydroxyamino)-2R-isobutylsuccinyl]-L-(4-oxyméthylcarboxy-β-alanine)phénylalanine-N-méthylamide ;
[4-(N-hydroxyamino)-2R-isobutylsuccinyl]-L-(4-oxyméthylcarboxyglycine)phénylalanine-N-méthylamide ;
[4-(N-hydroxyamino)-2R-isobutylsuccinyl]-L-(4-oxyméthylcarboxy-N-benzylamide)phénylalanine-N-méthylamide ;
[4-(N-hydroxyamino)-2R-isobutyl-3S-(2-thiénylthiométhyl)succinyl]-L-(4-oxyméthylcarboxyméthyl)phénylalanine-N-méthylamide ;
[4-(N-hydroxyamino)-2R-isobutyl-3S-(2-thiénylthiométhyl)succinyl]-L-(acide 4-oxyméthylcarboxylique)phénylalanine-N-méthylamide ;
[4-(N-hydroxyamino)-2R-isobutyl-3S-(2-thiénylthiométhyl)succinyl]-L-(4-oxyméthylcarboxyglycyl-méthyl-ester)phénylalanine-N-méthylamide ;
[4-(N-hydroxyamino)-2R-isobutyl-3S-(2-thiénylthiométhyl)succinyl]-L-(4-oxyméthylcarboxy-glycine)phénylalanine-N-méthylamide ;
[4-(N-hydroxyamino)-2R-isobutyl-3S-méthylsuccinyl]-L-(4-oxyméthylcarboxyglycyl-méthyl-ester)phénylalanine-N-méthylamide ;
[4-(N-hydroxyamino)-2R-isobutyl-3S-méthylsuccinyl]-L-(4-oxyméthylcarboxyglycine)phénylalanine-N-méthylamide ;
ou un sel d'un de ces composés.

10. Composé selon l'une quelconque des revendications 1 à 9, destiné à être utilisé en médecine humaine ou vétérinaire.

11. Utilisation d'un composé selon l'une quelconque des revendications 1 à 9, dans la préparation d'un agent destiné à traiter des maladies mettant en jeu la dégradation du collagène.

12. Composition pharmaceutique ou vétérinaire, comprenant un composé selon l'une quelconque des revendications 1 à 9 conjointement avec un véhicule acceptable d'un point de vue pharmaceutique et/ou vétérinaire.
